# EUROPÄISCHE PATENTSCHRIFT

(11) **EP 2 630 195 B1**
(45) Veröffentlichungstag und Bekanntmachung des Hinweises auf die Patenterteilung: **17.08.2016**
(21) Anmeldenummer: 11776392.0
(22) Anmeldetag: 18.10.2011
(51) Int. Cl.: C09B 11/28, G01N 33/58

(54) **NEUE AMIN-SUBSTITUIERTE TRICYCLISCHE FLUORESZENZFARBSTOFFE**
NEW AMINE-SUBSTITUTED TRICYCLIC FLUORESCENT DYES
COLORANTS FLUORESCENTS TRICYCLIC AMINO-SUBSTITUÉS

(30) Priorität: 19.10.2010 DE 102010042634
(43) Veröffentlichungstag der Anmeldung: 28.08.2013
(73) Patentinhaber: ATTO-TEC GmbH, 57076 Siegen (DE)
(72) Erfinder: ZILLES, Alexander, 57223 Kreuztal (DE); DREXHAGE, Karl-Heinz, 57076 Siegen (DE); KEMNITZER, Norbert Uwe, 57250 Netphen (DE); ARDEN-JACOB, Jutta, 90513 Zirndorf (DE); HAMERS-SCHNEIDER, Monika, 57258 Freudenberg (DE)
(74) Vertreter: Weickmann & Weickmann PartmbB
(86) Internationale Anmeldenummer: PCT/EP2011/068187
(87) Internationale Veröffentlichungsnummer: WO 2012/052435

(56) Entgegenhaltungen:
- DE-A1- 3 322 945
- GB-A- 420 743
- US-A1- 2006 211 029
- WU ET AL: "Fluorescent Amino- and Thiopyronin Dyes", ORGANIC LETTERS, AMERICAN CHEMICAL SOCIETY, US, Bd. 10, Nr. 9, 1. Januar 2008 (2008-01-01) , Seiten 1779-1782, XP009154809, ISSN: 1523-7060, DOI: 10.1021/OL800526S [gefunden am 2008-04-09]
- J.DEBRAY ET AL.: "Synthesis and Evaluation of fused Bipyrimidinacridines as novel pentacyclic Analogues of Quadruplex-Binder BRACO-19", ORG.BIOMOL.CHEM., Bd. 7, 2009, Seiten 5219-5228, XP002665746,
- J.SHI ET AL.: "Xanthenes: Fluorone Derivatives II", TETRAHEDRON LETT., Bd. 34, Nr. 38, 1993, Seiten 6013-6016, XP002665747,
- MARTINS ET AL: "Structure-based design of benzylamino-acridine compounds as G-quadruplex DNA telomere targeting agents", BIOORGANIC & MEDICINAL CHEMISTRY LETTERS, PERGAMON, ELSEVIER SCIENCE, GB, Bd. 17, Nr. 8, 30. März 2007 (2007-03-30), Seiten 2293-2298, XP022009252, ISSN: 0960-894X, DOI: 10.1016/J.BMCL.2007.01.056
- AHN ET AL: "Combinatorial Rosamine Library and Application to in Vivo Glutathione Probe", JOURNAL OF THE AMERICAN CHEMICAL SOCIETY, AMERICAN CHEMICAL SOCIETY, WASHINGTON, DC; US, Bd. 129, Nr. 15, 18. April 2007 (2007-04-18), Seiten 4510-4511, XP009154834, ISSN: 0002-7863
- K.MIZUKI ET AL.: "Fluorescence Enhancement of Bis-Acridine Orange Peptide, BAO, upon binding to double stranded DNA", ORG.BIOMOL.CHEM., Bd. 3, 2005, Seiten 578-580, XP002665748,

## Beschreibung

Die vorliegende Erfindung betrifft polycyclische Verbindungen der allgemeinen Formel (I), die sich durch einen Amin-Substituenten NR⁶R⁷ am zentralen Kohlenstoffatom des Chromophors auszeichnen, Verfahren zu ihrer Herstellung, sowie deren Verwendung zur Bestimmung von Analyten.

In der chemischen, medizinischen und biologischen Analytik werden organische Farbstoffe in vielfältiger Weise als Markierungs- bzw. Nachweisgruppen verwendet. Insbesondere Fluoreszenzfarbstoffe haben in neuerer Zeit große praktische Bedeutung gewonnen und andere frühere Verfahren, die etwa radioaktive Isotope verwenden, fast vollständig verdrängt.

Trotz der Verfügbarkeit einer großen Anzahl verschiedener Fluoreszenzfarbstoffe konnten bisher Probleme durch Hintergrundfluoreszenz, unspezifische Bindung, Instabilität unter intensiver Laser-Bestrahlung, etc. nicht befriedigend gelöst werden. Insbesondere ergeben sich häufig Probleme durch die - den meisten Farbstoffen eigene - geringe Verschiebung der Fluoreszenzbande relativ zur langwelligen Absorptionsbande. Sie beträgt typischerweise nur etwa 30 nm. Diese geringe, sogenannte Stokes'sche Verschiebung erschwert die saubere Trennung des Fluoreszenzsignals von gestreutem Anregungslicht.

Weiterhin ist es vielfach wünschenswert oder gar notwendig, mehrere Analyten bei Anregung mit derselben Lichtquelle anhand unterschiedlicher Fluoreszenz voneinander zu diskriminieren. Hierfür erforderliche Farbstoffe mit großer Stokes'scher Verschiebung, d.h. einer Verschiebung der Fluoreszenzbande relativ zur langwelligen Absorptionsbande von > 60 nm, und gleichzeitig hoher Fluoreszenzeffizienz sind nahezu gänzlich unbekannt.

Wu et al. (Org. Let. (2008), 10, 1779-1782) offenbart die Herstellung fluoreszierender Amino- und Thiopyroninfarbstoffe, wobei in Tabelle 2 Aminopyroninfarbstoffe mit einer Stoke'schen Verschiebung von bis zu 82 nm beschrieben werden.

Die der vorliegenden Erfindung zugrundeliegende Aufgabe bestand somit darin, Fluoreszenzfarbstoffe bereitzustellen, welche eine große Stokes'sche Verschiebung sowie eine hohe Fluoreszenzeffizienz aufweisen und zur qualitativen oder/und quantitativen Bestimmung von Analyten eingesetzt werden können. Weiterhin sollten diese Fluoreszenzfarbstoffe möglichst einfach und kostengünstig hergestellt werden können.

Diese Aufgabe wurde erfindungsgemäß gelöst durch polycyclische Verbindungen der allgemeinen Formel (I) 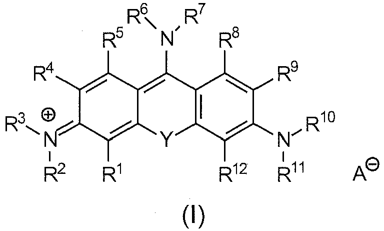 worin
R¹, R⁴, R⁵, R⁸, R⁹ und R¹² unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R², R³, R¹⁰ und R¹¹ unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R⁶ und R⁷ unabhängig voneinander Wasserstoff, NR¹³R¹⁴, eine Kohlenwasserstoffgruppe mit 1-20 C-Atomen, oder eine Analytmolekülgruppe bedeutet, wobei R¹³ und R¹⁴ unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeutet, und wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst, oder
R⁶ und R⁷ zusammen mit dem N-Atom, an welches sie gebunden sind, einen 3- bis 7-gliedrigen Ring bilden, wobei der Ring eine oder mehrere Doppelbindungen oder/und ein oder mehrere zusätzliche Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
Y CR¹⁵R¹⁶ bedeutet, wobei R¹⁵ und R¹⁶ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst, oder
R¹⁵ und R¹⁶ zusammen mit dem C-Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst, und
A⁻ein Anion bedeutet,
oder worin R¹ und R¹, R³ und R⁴, R⁹ und R¹⁰ oder/und R¹¹ und R¹² zusammen mit den Atomen, an welche sie gebunden sind, einen 5- oder 6-gliedrigen Ring bilden, wobei der Ring gegebenenfalls eine oder mehrere Doppelbindungen oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfasst, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst.

Der Begriff "Kohlenwasserstoffgruppe", wie er im Rahmen der vorliegenden Anmeldung verwendet wird, umfasst einen gesättigten oder ungesättigten Kohlenwasserstoffrest, welcher linear, verzweigt oder cyclisch ausgebildet sein kann und eine Bindungsvalenz an einem beliebigen der 1-20 Kohlenstoffatome aufweist. Beispiele für Kohlenwasserstoffgruppen im Sinne der vorliegenden Erfindung umfassen Alkyl, Cycloalkyl, Alkenyl, Alkinyl und Aryl. Sofern die Kohlenwasserstoffgruppe ein oder mehrere Heteroatome ausgewählt aus der Gruppe bestehend aus N, O und S umfasst, so kann die Kohlenwasserstoffgruppe zudem als Heteroaryl ausgebildet sein.

Der Begriff "Alkyl", wie er im Rahmen der vorliegenden Anmeldung verwendet wird, bezeichnet einen gesättigten, linearen oder verzweigten Kohlenwasserstoffrest mit 1-20 Kohlenstoffatomen, welcher eine Bindungsvalenz an einem beliebigen der 1-20 Kohlenstoffatome aufweist. Bevorzugt stellt Alkyl einen Kohlenwasserstoffrest mit 1-12 Kohlenstoffatomen, stärker bevorzugt mit 1-6 Kohlenstoffatomen, dar. Besonders bevorzugte Alkyle umfassen Methyl, Ethyl, n-Propyl, iso-Propyl, n-Butyl, iso-Butyl, sec-Butyl und tert-Butyl.

Der Begriff "Cycloalkyl", wie er im Rahmen der vorliegenden Anmeldung verwendet wird, bezeichnet einen gesättigten oder ungesättigten, cyclischen Kohlenwasserstoffrest mit 3-20 Kohlenstoffatomen, welcher eine Bindungsvalenz an einem beliebigen der 3-20 Kohlenstoffatome aufweist. Bevorzugt stellt Cycloalkyl einen cyclischen Kohlenwasserstoffrest mit 3-12 Kohlenstoffatomen, stärker bevorzugt mit 3-8 Kohlenstoffatomen, dar. Besonders bevorzugte Cycloalkyle umfassen Cyclopropyl, Cyclobutyl, Cyclopentyl, Cyclohexyl, Cycloheptyl und Cyclooctyl.

Der Begriff "Alkenyl", wie er im Rahmen der vorliegenden Anmeldung verwendet wird, bezeichnet einen ungesättigten, linearen oder verzweigten Kohlenwasserstoffrest mit 2-20 Kohlenstoffatomen, welcher eine Bindungsvalenz an einem beliebigen der 2-20 Kohlenstoffatome und mindestens eine Doppelbindung aufweist. Bevorzugt stellt Alkenyl einen Kohlenwasserstoffrest mit 2-12 Kohlenstoffatomen, stärker bevorzugt mit 2-6 Kohlenstoffatomen, dar. Besonders bevorzugte Alkenyle umfassen Ethenyl, Propenyl und Butenyl.

Der Begriff "Alkinyl", wie er im Rahmen der vorliegenden Anmeldung verwendet wird, bezeichnet einen ungesättigten, linearen oder verzweigten Kohlenwasserstoffrest mit 2-20 Kohlenstoffatomen, welcher eine Bindungsvalenz an einem beliebigen der 2-20 Kohlenstoffatome und mindestens eine Dreifachbindung aufweist. Bevorzugt stellt Alkinyl einen Kohlenwasserstoffrest mit 2-12 Kohlenstoffatomen, stärker bevorzugt mit 2-6 Kohlenstoffatomen, dar. Besonders bevorzugte Alkinyle umfassen Ethinyl, Propinyl und Butinyl.

Der Begriff "Aryl", wie er im Rahmen der vorliegenden Anmeldung verwendet wird, bezeichnet ein aromatisches Ringsystem mit 3-20 Ringatomen, stärker bevorzugt mit 6-14 Ringatomen, welches als Ringatome ausschließlich Kohlenstoffatome enthält und eine Bindungsvalenz an einem beliebigen Kohlenstoffatom der 3-20 ringbildenden Atome aufweist. Bevorzugte Aryle umfassen Phenyl, Naphthyl, Anthracenyl und Phenanthrenyl.

Der Begriff "Heteroaryl", wie er im Rahmen der vorliegenden Anmeldung verwendet wird, bezeichnet ein aromatisches Ringsystem mit 3-20 Ringatomen, stärker bevorzugt mit 5-14 Ringatomen, welches als Ringatome neben Kohlenstoffatomen mindestens ein Heteroatom ausgewählt aus der Gruppe bestehend aus N, O und S enthält und eine Bindungsvalenz an einem beliebigen Kohlenstoffatom oder Stickstoffatom der 3-20 ringbildenden Atome aufweist. Bevorzugte Heteroaryle umfassen Furanyl, Imidazolyl, Isothiazolyl, Isoxazolyl, Oxadiazolyl, Oxazolyl, Pyrazolyl, Pyrrolyl, Tetrazolyl, Thiadiazolyl, Thiazolyl, Thienyl, Triazolyl, Pyrazinyl, Pyridazinyl, Pyridyl, Pyrimidinyl und Triazinyl.

Der Begriff "Halogen", wie er im Rahmen der vorliegenden Anmeldung verwendet wird, umfasst Fluor, Chlor, Brom und lod.

Der Begriff "Analytmolekülgruppe", wie er im Rahmen der vorliegenden Anmeldung verwendet wird, bezeichnet den Rest Z eines Analytmoleküls, welches durch die allgemeine Formel Z-NH₂ dargestellt werden kann und insofern mindestens eine freie Aminogruppe zur Ausbildung einer kovalenten Bindung mit einem geeigneten Reaktionspartner aufweist. In einer bevorzugten Ausführungsform der Erfindung handelt es sich bei dem Analytmolekül um ein Biomolekül, insbesondere um ein Biomolekül ausgewählt aus der Gruppe bestehend aus einem Peptid, einem Polypeptid, einem Protein, einem Nukleotid, einem Polynukleotid, einem Nukleosid, einer Nukleinsäure, einem Nukleinsäureanalogon und einem Hapten.

In den erfindungsgemäßen polycylischen Verbindungen der allgemeinen Formel (I) bilden R¹ und R², R³ und R⁴, R⁹ und R¹⁰ oder/und R¹¹ und R¹² zusammen mit den Atomen, an welche sie gebunden sind, in einer bevorzugten Ausführungsform einen 5- oder 6-gliedrigen Ring, wobei 6-gliedrige Ringe stärker bevorzugt sind. Der mindestens eine 5- oder 6-gliedrige Ring kann dabei eine oder mehrere Doppelbindungen oder/und einen oder mehrere Substituenten, beispielsweise 1, 2, 3 oder 4 Substituenten, umfassen, welche ihrerseits gleich oder verschieden sein können. Die Fluoreszenz derartiger Verbindungen ist besonders effizient und zeigt lediglich eine geringe Abhängigkeit von Temperatur und Umwelteinflüssen.

Sofern der mindestens eine 5- oder 6-gliedrige Ring einen oder mehrere Substituenten umfasst, so handelt es sich dabei um Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst.

Besonders bevorzugt gelangen als Substituenten des mindestens einen 5-oder 6-gliedrigen Ringes Kohlenwasserstoffgruppen mit 1-20 C-Atomen, insbesondere Alkylgruppen mit 1-6 C-Atomen, zum Einsatz, wobei die Kohlenwasserstoffgruppen im Falle einer Substitution vorzugsweise einen oder mehrere Substituenten ausgewählt aus der Gruppe bestehend aus COOH, COO(Alkyl), COO(Aryl) und SO₃H umfassen.

In einer weiteren bevorzugten Ausführungsform der Erfindung stellen die Substituenten R², R³, R¹⁰ oder/und R¹¹ in den polycylischen Verbindungen der allgemeinen Formel (I) eine Kohlenwasserstoffgruppe mit 1-20 C-Atomen dar, wobei die Kohlenwasserstoffgruppe gegebenenfalls einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst. Besonders bevorzugt handelt es sich bei der Kohlenwasserstoffgruppe um eine Alkylgruppe mit 1-6 C-Atomen, insbesondere um Methyl oder Ethyl.

In einer weiteren bevorzugten Ausführungsform der Erfindung bezeichnen mindestens zwei Reste ausgewählt aus der Gruppe bestehend aus R⁵, R⁸ und R¹², insbesondere R⁵ und R⁸, Wasserstoff. Besonders bevorzugt gelangen erfindungsgemäß indessen polycylische Verbindungen der allgemeinen Formel (I) zum Einsatz, in welchen R⁵, R⁸ und R¹² jeweils Wasserstoff darstellen.

Die erfindungsgemäßen polycylischen Verbindungen der allgemeinen Formel (I) umfassen obligatorisch eine funktionelle Gruppe NR⁶R⁷. Vorzugsweise bedeuten R⁶ und R⁷ hierbei unabhängig voneinander Wasserstoff oder eine Kohlenwasserstoffgruppe mit 1-20 C-Atomen, wobei die Kohlenwasserstoffgruppe gegebenenfalls einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst. Stärker bevorzugt repräsentieren R⁶ und R⁷ hierbei nicht gleichzeitig Wasserstoff.

Noch stärker bevorzugt stellt mindestens einer der Substituenten R⁶ und R⁷, d.h. R⁶ oder/und R⁷, eine Kohlenwasserstoffgruppe mit 1-20 C-Atomen, insbesondere eine Alkylgruppe mit 1-6 C-Atomen oder eine Arylgruppe mit 6-14 Ringatomen, dar, wobei die Kohlenwasserstoffgruppe im Falle einer Substitution vorzugsweise einen oder mehrere Substituenten ausgewählt aus der Gruppe bestehend aus Halogen, OH, SH, NH₂, COOH, COO(Alkyl), COO(Aryl) und SO₃H, insbesondere einen oder mehrere Substituenten ausgewählt aus der Gruppe bestehend aus OH, NH₂, COOH und SO₃H, umfasst. Besonders bevorzugt bezeichnet einer der Substituenten R⁶ und R⁷ ein Wasserstoffatom, während der andere Substituent vorstehend definierte Kohlenwasserstoffgruppe mit 1-20 C-Atomen darstellt.

In den erfindungsgemäßen polycylischen Verbindungen der allgemeinen Formel (I) bedeutet Y CR¹⁵R¹⁶, wobei vorzugsweise mindestens einer der Reste R¹⁵ und R¹⁶ eine Kohlenwasserstoffgruppe mit 1-20 C-Atomen, bevorzugt eine Alkylgruppe mit 1-6 C-Atomen, stärker bevorzugt Methyl oder Ethyl, darstellt.

Tabelle 1 zeigt exemplarisch besonders bevorzugte polycyclische Verbindungen der allgemeinen Formel (I) mit Carbopyroninstruktur.

**Tabelle 1**

| Verbindung | Struktur | λ_{abs} [nm] | λ_{fl} [nm] | η_{fl} [%] | Stokes Shift [nm] |
|---|---|---|---|---|---|
| **1** | 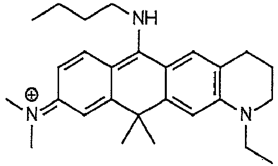 | 472 EtOH | 586 EtOH | 75 EtOH | 114 EtOH |
| AZ 274 | | | | | |
| **2** | 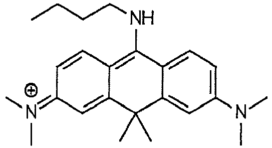 | 460 EtOH | 580 EtOH | 68 EtOH | 120 EtOH |
| AZ 279 | | 453 PBS | 606 PBS | 34 PBS | 153 PBS |
| **3** | 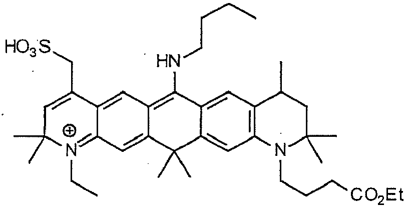 | 485 EtOH | 617 EtOH | 67 EtOH | 132 EtOH |
| AZ 281 | | 484 PBS | 650 PBS | 31 PBS | 166 PBS |
| **4** | 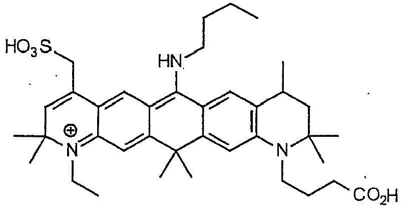 | 484 EtOH | 623 EtOH | 65 EtOH | 139 EtOH |
| AZ 285 | | 484 PBS | 653 PBS | 32 PBS | 169 PBS |
| **5** AZ 286 | 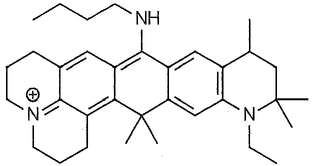 | 480 EtOH | 601 EtOH | 79 EtOH | 121 EtOH |
| **6** | 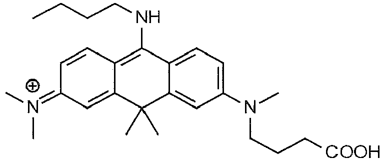 | 465 EtOH | 579 EtOH | 65 EtOH | 114 EtOH |
| AZ 291 | | 455 PBS | 605 PBS | | 150 PBS |
| **7** | 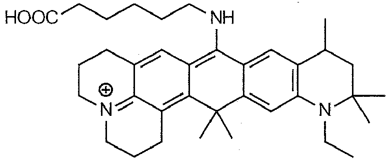 | 480 EtOH | 601 EtOH | 78 EtOH | 121 EtOH |
| AZ 292 | | 476 PBS | 618 PBS | | 142 PBS |
| **8** | 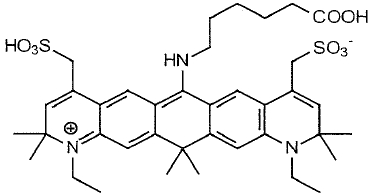 | 491 EtOH | 651 EtOH | 61 EtOH | 160 EtOH |
| AZ 312 | | 490 PBS | 670 PBS | 26 PBS | 180 PBS |
| **9** | 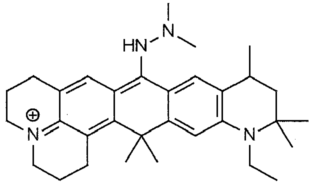 | 492 | 605 | 50 | 113 |
| AZ 315 | | EtOH | EtOH | EtOH | EtOH |
| **10** | 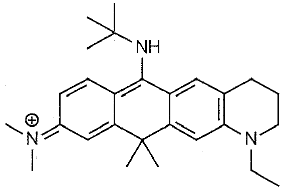 | 493 EtOH | 644 EtOH | 63 EtOH | 151 EtOH |
| AZ 317 | | | | | |
| **11** | 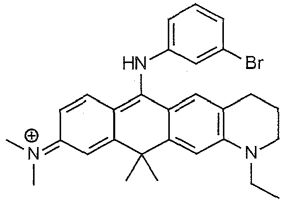 | 523 EtOH | 644 EtOH | 1 EtOH | 121 EtOH |
| AZ 322 | | | | | |
| **12** | 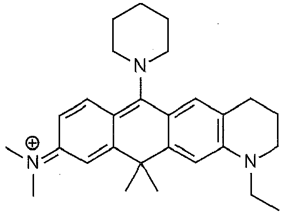 | 475 EtOH | 588 EtOH | 8 EtOH | 113 EtOH |
| AZ 323 | | | | | |
| **13** | 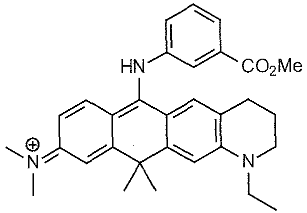 | 520 EtOH | 641 EtOH | 2 EtOH | 121 EtOH |
| AZ 324 | | | | | |
| **14** | 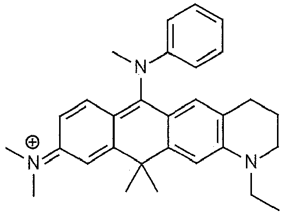 | 629 EtOH | | < 0.1 EtOH | |
| AZ 327 | | | | | |
| **15** | 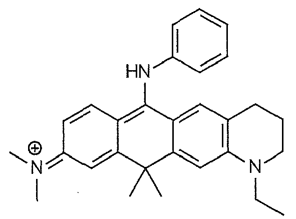 | 507 EtOH | 620 EtOH | < 1 EtOH | 113 EtOH |
| AZ 329 | | | | | |
| **16** | 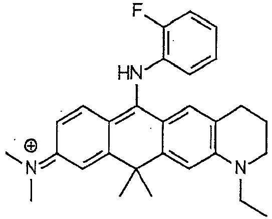 | 512 EtOH | 620 EtOH | 4 EtOH | 108 EtOH |
| AZ 330 | | | | | |
| **17** | 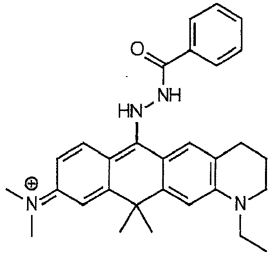 | 497 EtOH | 622 EtOH | 55 EtOH | 125 EtOH |
| AZ 332 | | | | | |
| **18** | 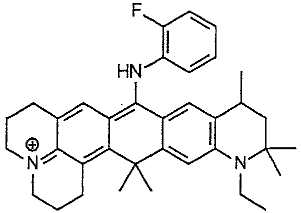 | 526 EtOH | 627 EtOH | 11 EtOH | 101 EtOH |
| AZ 334 | | | | | |
| **19** | 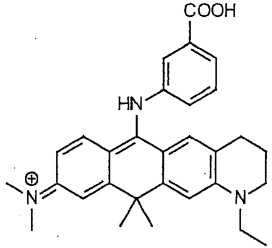 | 517 EtOH | 638 EtOH | 2 EtOH | 121 EtOH |
| AZ 336 | | | | | |
| **20** | 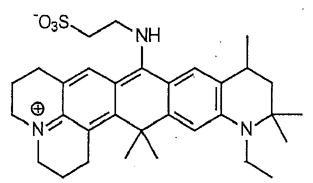 | 484 EtOH | 603 EtOH | 78 EtOH | 119 EtOH |
| AZ 342 | | | | | |

Tabelle 2 zeigt exemplarisch polycyclische Verbindungen der allgemeinen Formel (I) mit Rhodaminstruktur als Vergleichsbeispiele.

**Tabelle 2 (Vergleichsbeispiele)**

| Verbindung | Struktur | λ_{abs} [nm] | λ_{fl} [nm] | η_{fl} [%] | Stokes Shift [nm] |
|---|---|---|---|---|---|
| **21** | 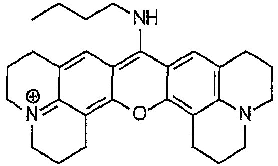 | 456 EtOH | 555 EtOH | 82 EtOH | 99 EtOH |
| AZ 277 | | | | | |
| **22** | 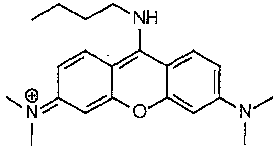 | 433 EtOH | 524 EtOH | 47 EtOH | 91 EtOH |
| AZ 278 | | | | | |
| **23** | 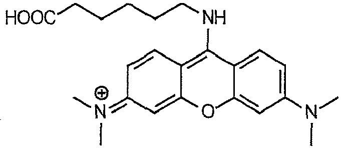 | 434 EtOH | 524 EtOH | 48 EtOH | 90 EtOH |
| AZ 293 | | | | | |
| **24** | 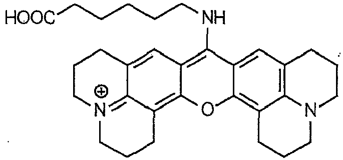 | 459 EtOH | 555 EtOH | 80 EtOH | 96 EtOH |
| AZ 294 | | 454 PBS | 568 PBS | 60 PBS | 114 PBS |
| **25** | 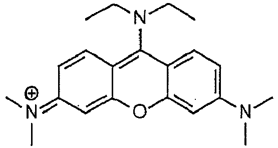 | 484 | 546 EtOH | | 62 EtOH |
| AZ 313 | | EtOH | | | |

Tabelle 3 zeigt exemplarisch weitere polycyclische Verbindungen der allgemeinen Formel (I) als Vergleichsbeispiele.

**Tabelle 3 (Vergleichsbeispiele)**

| Verbindung | Struktur | λ_{abs} [nm] | λ_{fl} [nm] | η_{fl} [%] | Stokes Shift [nm] |
|---|---|---|---|---|---|
| **26** | 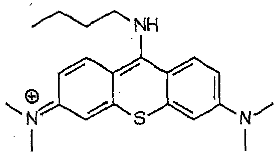 | 451 EtOH | 563 EtOH | 55 EtOH | 112 EtOH |
| AZ 307 | | | | | |
| **27** | 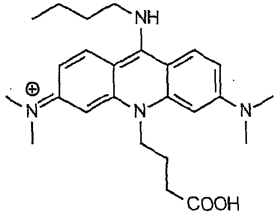 | 432 EtOH | 506 EtOH | | 74 EtOH |
| AZ 308 | | | | | |

In einem weiteren Aspekt betrifft die Erfindung ein Verfahren zur Herstellung einer polycylischen Verbindung der allgemeinen Formel (I), wobei eine polycyclische Vorläuferverbindung der allgemeinen Formel (II) 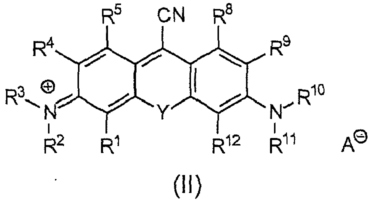 oder eine polycyclische Vorläuferverbindung der allgemeinen Formel (III) 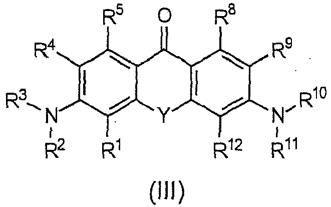 mit einem primären oder sekundären Amin der allgemeinen Formel HNR⁶R⁷ umgesetzt wird und R¹, R², R³, R⁴, R⁵, R⁶, R⁷, R⁸, R⁹, R¹⁰, R¹¹, R¹², Y und A⁻ wie für die erfindungsgemäßen, oben beschriebenen polycyclischen Verbindungen der allgemeinen Formel (I) definiert sind.

Die Herstellung der erfindungsgemäßen polycyclischen Verbindungen der allgemeinen Formel (I) erfolgt üblicherweise in Gegenwart eines Lösungsmittels, insbesondere eines Lösungsmittels ausgewählt aus der Gruppe bestehend aus Aceton, Acetonitril, Chloroform, Dichlormethan, Dimethylsulfoxid und N,N-Dimethylformamid. Sofern erforderlich, können auch Gemische vorstehend genannter Lösungsmittel zum Einsatz gelangen.

Im einzelnen wird zur Herstellung der polycyclischen Verbindungen der allgemeinen Formel (I) eine polycyclische Vorläuferverbindung der allgemeinen Formel (II) oder (III) beispielsweise in einem Lösungsmittel, wie es oben definiert ist, gelöst, mit einem primären oder sekundären Amin der allgemeinen Formel HNR⁶R⁷ versetzt, das Reaktionsgemisch bei einer Temperatur von üblicherweise mindestens 0°C, insbesondere bei einer Temperatur im Bereich von 15°C bis 35°C, bis zur Beendigung der Reaktion gerührt, und das Reaktionsprodukt unter Verwendung von dem Fachmann bekannten Methoden gereinigt.

In einer Ausführungsform kann die Herstellung der polycyclischen Verbindungen der allgemeinen Formel (I), insbesondere bei Einsatz einer polycyclischen Vorläuferverbindung der allgemeinen Formel (III) als Ausgangsmaterial, zusätzlich eine Aktivierung der polycyclischen Vorläuferverbindung umfassen, welche üblicherweise vor Inkontaktbringen der polycyclischen Vorläuferverbindung mit dem primären oder sekundären Amin der allgemeinen Formel HNR⁶R⁷ durchgeführt wird. Zur Aktivierung der polycyclischen Vorläuferverbindungen der allgemeinen Formel (II) oder (III) kann grundsätzlich jedes dem Fachmann geeignet erscheinende Aktivierungsreagenz verwendet werden, wobei chemische Aktivierungsreagenzien wie beispielsweise Trifluormethansulfonsäureanhydrid als bevorzugt gelten.

Weiterhin beschrieben werden hierin polycylischen Vorläuferverbindungen der allgemeinen Formel (II) 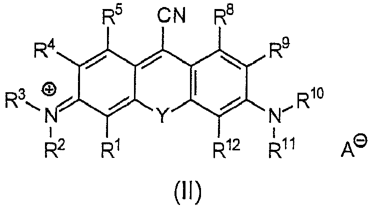 worin R¹, R², R³, R⁴, R⁵, R⁸, R⁹, R¹⁰, R¹¹, R¹², Y und A⁻ wie für die erfindungsgemäßen, oben beschriebenen polycyclischen Verbindungen der allgemeinen Formel (I) definiert sind. Was bevorzugte Ausführungsformen der in den polycylischen Vorläuferverbindungen der allgemeinen Formel (II) enthaltenen Substituenten betrifft, so wird auf die Erläuterungen in Zusammenhang mit den polycyclischen Verbindungen der allgemeinen Formel (I) verwiesen.

Tabelle 4 zeigt exemplarisch besonders bevorzugte polycyclische Vorläuferverbindungen der allgemeinen Formel (II) mit ihren Absorptions-und Fluoreszenzmaxima (gemessen in Ethanol).

**Tabelle 4**

| Verbindung | Struktur | λ_{abs} [nm] | λ_{fl} [nm] |
|---|---|---|---|
| **28** | 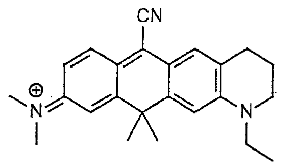 | 723 | 755 |
| AZ 111 | | | |
| **29** | 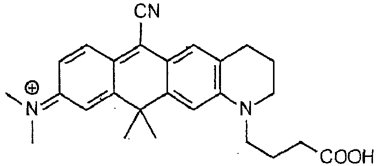 | 726 | 754 |
| AZ 112 | | | |
| **30** | 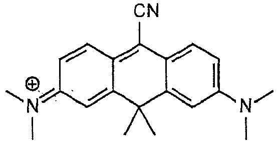 | 710 | 742 |
| AZ 115 | | | |
| **31** | 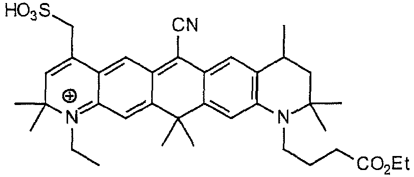 | 771 | 794 |
| AZ 144 | | | |
| **32** | 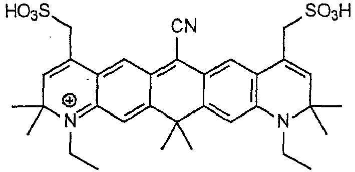 | 801 | 830 |
| AZ 147 | | | |
| **33** | 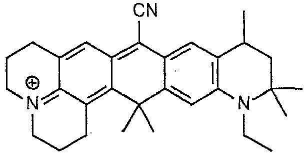 | 741 | 766 |
| AZ 284 | | | |
| **34** | 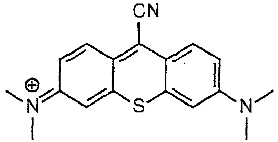 | 666 | 690 |
| AZ 305 | | | |
| **35** | 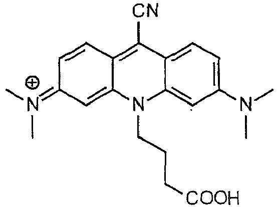 | 583 | 599 |
| AZ 306 | | | |

Bei den Verbindungen 34 und 35 handelt es sich um Vergleichsbeispiele. Weiterhin beschrieben wird hierin ein Verfahren zur Herstellung einer polycylischen Vorläuferverbindung der allgemeinen Formel (II), wobei eine polycyclische Vorläuferverbindung der allgemeinen Formel (IV) 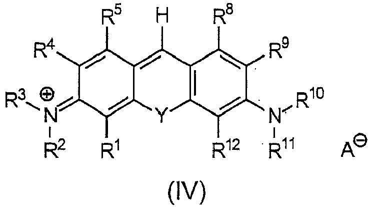 mit einem Tetraalkylammoniumcyanid der allgemeinen Formel N(Alkyl)₄CN und einem Oxidationsmittel umgesetzt wird und R¹, R², R³, R⁴, R⁵, R⁸, R⁹, R¹⁰, R¹¹, R¹² und Y wie für die erfindungsgemäßen, oben beschriebenen polycyclischen Verbindungen der allgemeinen Formel (I) definiert sind.

Die Herstellung der polycyclischen Vorläuferverbindungen der allgemeinen Formel (II) erfolgt vorzugsweise in Gegenwart eines Lösungsmittels, insbesondere eines Lösungsmittels ausgewählt aus der Gruppe bestehend aus Aceton, Acetonitril, Chloroform, Dichlormethan, Dimethylsulfoxid und N,N-Dimethylformamid. Sofern erforderlich, können auch Gemische vorstehend genannter Lösungsmittel zum Einsatz gelangen.

Im einzelnen wird zur Herstellung der polycyclischen Vorläuferverbindungen der allgemeinen Formel (II) eine polycyclische Vorläuferverbindung der allgemeinen Formel (IV) beispielsweise in einem Lösungsmittel, wie es oben definiert ist, gelöst, mit einem Tetraalkylammoniumcyanid der allgemeinen Formel N(Alkyl)₄CN und einem geeigneten Oxidationsmittel versetzt, das Reaktionsgemisch bei einer Temperatur von üblicherweise mindestens 0°C, insbesondere bei einer Temperatur im Bereich von 15°C bis 35°C, bis zur Beendigung der Reaktion gerührt, und das Reaktionsprodukt unter Verwendung von dem Fachmann bekannten Methoden gereinigt.

Bei dem Oxidationsmittel kann es sich grundsätzlich um ein beliebiges Reagenz handeln, welches eine Oxidation zum Chromophor bewirken kann. Für die Zwecke der vorliegenden Erfindung hat sich Chloranil als besonders vorteilhaft erwiesen, wobei auch andere dem Fachmann geeignet erscheinende Oxidationsmittel, wie beispielsweise Natriumperiodat oder Bleidioxid, zur Anwendung gelangen können.

In noch einem weiteren Aspekt betrifft die Erfindung polycylische Vorläuferverbindungen der allgemeinen Formel (III) 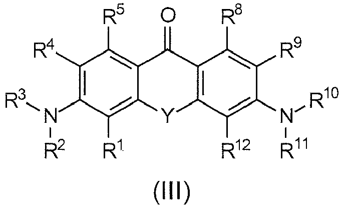 worin R¹, R², R³, R⁴, R⁵, R⁸, R⁹, R¹⁰, R¹¹, R¹² und Y wie für die erfindungsgemäßen, oben beschriebenen polycyclischen Verbindungen der allgemeinen Formel (I) definiert sind. Was bevorzugte Ausführungsformen der in den polycylischen Vorläuferverbindungen der allgemeinen Formel (III) enthaltenen Substituenten betrifft, so wird auf die Erläuterungen in Zusammenhang mit den polycyclischen Verbindungen der allgemeinen Formel (I) verwiesen.

Tabelle 5 zeigt exemplarisch besonders bevorzugte polycyclische Vorläuferverbindungen der allgemeinen Formel (III) mit ihren Absorptionsmaxima (gemessen in Ethanol).

**Tabelle 5**

| Verbindung | Struktur | λ_{abs} [nm] |
|---|---|---|
| **36** | 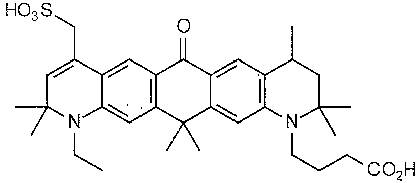 | 421 |
| AZ 35-A | | |
| **37** | 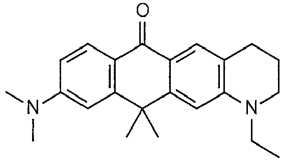 | 400 |
| AZ 110-A | | |
| **38** | 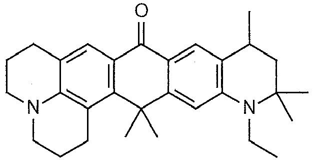 | 409 |
| AZ 124-A | | |
| **39** | 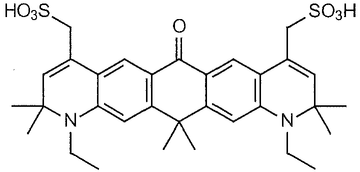 | 438 |
| AZ 145-A | | |
| **40** | 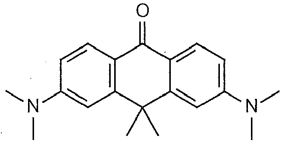 | 394 |
| AZ 151-A | | |
| **41** | 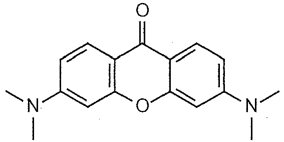 | 380 |
| AZ 156-A | | |
| **42** | 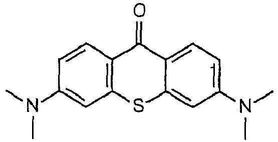 | 384 |
| AZ 170-A | | |
| **43** | 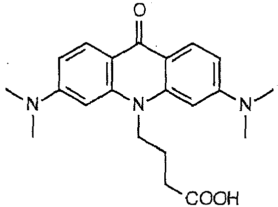 | 360 |
| ATTO 495-A | | |
| **44** | 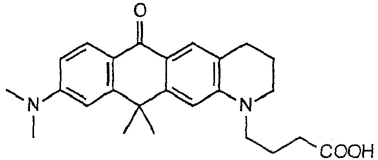 | 401 |
| ATTO 610-A | | |

Bei den Verbindungen 41, 42 und 43 handelt es sich um Vergleichsbeispiele. In noch einem weiteren Aspekt betrifft die Erfindung ein Verfahren zur Herstellung einer polycylischen Vorläuferverbindung der allgemeinen Formel (III), wobei eine polycyclische Vorläuferverbindung der allgemeinen Formel (IV) 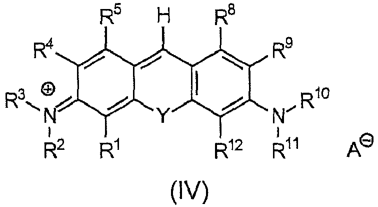 mit einem Oxidationsmittel umgesetzt wird und R¹, R², R³, R⁴, R⁵, R⁸, R⁹, R¹⁰, R¹¹, R¹², Y und A⁻ wie für die erfindungsgemäßen, oben beschriebenen polycyclischen Verbindungen der allgemeinen Formel (I) definiert sind.

Die Herstellung der erfindungsgemäßen polycyclischen Vorläuferverbindungen der allgemeinen Formel (III) erfolgt vorzugsweise in Gegenwart eines Lösungsmittels, insbesondere eines Lösungsmittels ausgewählt aus der Gruppe bestehend aus Aceton, Acetonitril, Chloroform, Dichlormethan, Dimethylsulfoxid und N,N-Dimethylformamid. Sofern erforderlich, können auch Gemische vorstehend genannter Lösungsmittel zum Einsatz gelangen.

Im einzelnen wird zur Herstellung der polycyclischen Vorläuferverbindungen der allgemeinen Formel (III) eine polycyclische Vorläuferverbindung der allgemeinen Formel (IV) beispielsweise in einem Lösungsmittel, wie es oben definiert ist, gelöst, mit einem Oxidationsmittel versetzt, das Reaktionsgemisch bei einer Temperatur von üblicherweise mindestens 0°C, insbesondere bei einer Temperatur im Bereich von 15°C bis 35°C, bis zur Beendigung der Reaktion gerührt, und das Reaktionsprodukt unter Verwendung von dem Fachmann bekannten Methoden gereinigt.

Bei dem Oxidationsmittel kann es sich grundsätzlich um ein beliebiges Reagenz handeln, welches eine regioselektive Oxidation am zentralen Kohlenstoffatom des Chromophors bewirken kann. Für die Zwecke der vorliegenden Erfindung hat sich Kaliumpermanganat als besonders vorteilhaft erwiesen, wobei auch andere dem Fachmann geeignet erscheinende Oxidationsmittel zur Anwendung gelangen können.

Tabelle 6 zeigt exemplarisch besonders bevorzugte polycyclische Vorläuferverbindungen der allgemeinen Formel (IV) mit ihren Absorptions-und Fluoreszenzmaxima (gemessen in Ethanol).

**Tabelle 6**

| Verbindung | Struktur | λ_{abs} [nm] | λ_{fl} [nm] |
|---|---|---|---|
| **45** | 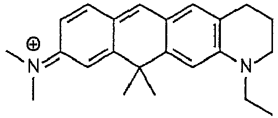 | 616 | 635 |
| AZ 110 | | | |
| **46** | 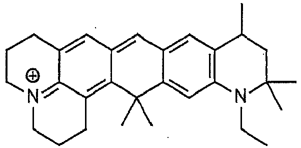 | 628 | 649 |
| AZ 124 | | | |
| **47** | 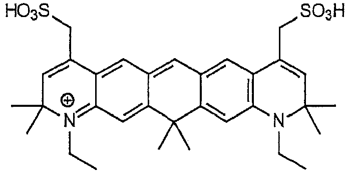 | 665 | 687 |
| AZ 145 | | | |
| **48** | 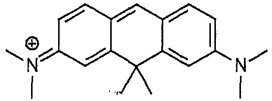 | 605 | 628 |
| AZ 151 | | | |
| **49** | 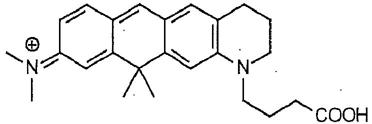 | 616 | 636 |
| ATTO 610 | | | |

In noch einem weiteren Aspekt betrifft die Erfindung die Verwendung einer erfindungsgemäßen polycyclischen Verbindung der allgemeinen Formel (I) oder einer erfindungsgemäßen polycyclischen Vorläuferverbindung der allgemeinen Formel (III) zur qualitativen oder/und quantitativen Bestimmung eines Analyten in einer Probe, wobei es sich bei dem Analyten um eine beliebige biologische oder chemische Substanz handeln kann, die photochemisch nachweisbar ist. Bevorzugte Analyten im Sinne der vorliegenden Anmeldung sind Biomoleküle, insbesondere Peptide, Polypeptide, Proteine, Nukleotide, Polynukleotide, Nukleoside, Nukleinsäuren, Nukleinsäureanaloga oder Haptene.

Der Analyt kann grundsätzlich aus einer beliebigen Quelle stammen, ist jedoch bevorzugt in einem Körperfluid, wie beispielsweise Vollblut, Plasma, Serum, Lymphflüssigkeit, Gallenflüssigkeit, Cerebrospinalflüssigkeit, extrazelluläre Gewebeflüssigkeit, Harn, Speichel und Schweiß, in einer Abwasserprobe oder in einem Lebensmittel enthalten. Bevorzugt wird mittels der hierin beschriebenen polycyclischen Verbindungen oder Vorläuferverbindungen allerdings das Vorhandensein oder/und die Menge eines Analyten in einer Probe aus Vollblut, Plasma, Serum oder Urin bestimmt.

Ein Vorteil der erfindungsgemäßen polycyclischen Verbindungen der allgemeinen Formel (I) bzw. der polycyclischen Vorläuferverbindungen der allgemeinen Formeln (II) und (III) ist, dass durch die Substituentenvariation die physikalischen und chemischen Eigenschaften der Verbindungen, wie beispielsweise die Lage der Absorptions- und Fluoreszenzmaxima, die Löslichkeit, die Fluoreszenz-Quantenausbeute sowie die Abklingzeit, stark variiert und somit den jeweiligen Anforderungen angepasst werden können. Auf diese Weise können beispielsweise Interferenzen mit Störsubstanzen in Proben wie Serum, Blut oder Plasma vermindert oder sogar gänzlich vermieden werden.

Um eine Bestimmung des Analyten zu ermöglichen, weisen die erfindungsgemäßen polycyclischen Verbindungen oder Vorläuferverbindungen neben der obligatorisch vorhandenen Gruppierung -NR⁶R⁷ vorzugsweise mindestens eine zur kovalenten Kopplung befähigte funktionelle Gruppe, wie beispielsweise OH, SH, NH₂ oder/und COOH, auf. Über diese Kopplungsgruppe, welche sich an einer beliebigen Position des Moleküls befinden kann, kann die jeweilige Verbindung mittels bekannter Verfahren, z.B. über die Zwischenstufe eines N-Hydroxysuccinimidylesters, an einen geeigneten Träger oder/und einen Analyten, wie er oben beschrieben ist, gekoppelt werden.

Sofern der Analyt durch die allgemeine Formel Z-NH₂ darstellbar ist und damit mindestens eine freie Aminogruppe umfasst, so kann der Analyt auch direkt mit einer polycyclischen Vorläuferverbindung der allgemeinen Formel (II) oder (III) zur Reaktion gebracht werden. Auf diese Weise können polycyclische Verbindungen der allgemeinen Formel (I) dargestellt werden, in welchen die Gruppierung -NR⁶R⁷ unmittelbar dem Analyten entstammt. In diesem Fall ist es nicht erforderlich, dass die erfindungsgemäßen polycyclischen Verbindungen oder Vorläuferverbindungen eine zusätzliche, zur kovalenten Kopplung an den Analyten befähigte funktionelle Gruppe umfassen.

Gelangt im Rahmen der Bestimmung des Analyten ein Träger zum Einsatz, so kann der Träger grundsätzlich aus jedem dem Fachmann geeignet erscheinenden Material bestehen, das von der zu untersuchenden Probe benetzt werden kann. Beispiele für derartige Trägermaterialien umfassen beispielsweise poröses Glas, Kunststoffe, Ionenaustauschharze, Dextrane, Cellulose, Cellulosederivate oder/und hydrophile Polymere, sind jedoch nicht auf diese beschränkt.

In einer bevorzugten Ausführungsform der Erfindung sieht die Bestimmung des Analyten indessen eine Markierung des Analyten durch kovalente Bindung einer erfindungsgemäßen polycyclischen Verbindung der allgemeinen Formel (I) bzw. einer polycyclischen Vorläuferverbindung der allgemeinen Formel (II) oder (III) an den Analyten vor.

Tabelle 7 zeigt exemplarisch biologische Konjugate, welche durch Kopplung an besonders bevorzugte polycyclische Verbindungen der allgemeinen Formel (I) erhalten werden können.

| Verbindung | Struktur | λ_{abs} [nm] | λ_{fl} [nm] | η_{fl} [%] | Stokes Shift [nm] |
|---|---|---|---|---|---|
| **50** | 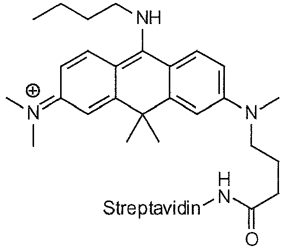 | 463 PBS | 592 PBS | 17 PBS | 129 PBS |
| AZ 291-Streptavidin | | | | | |
| **51** | 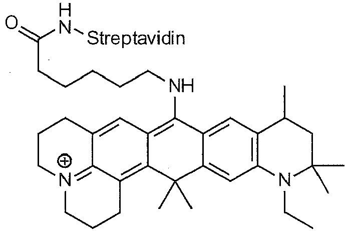 | 483 PBS | 618 PBS | 50 PBS | 135 PBS |
| AZ 292-Streptavidin | | | | | |

Zur qualitativen oder/und quantitativen Bestimmung des Analyten können grundsätzlich alle aus dem Stand der Technik bekannten Methoden eingesetzt werden, sofern diese ein messbares Signal erzeugen, das manuell oder unter Verwendung geeigneter Mittel ausgewertet werden kann. Im Rahmen der vorliegenden Erfindung gelangen bevorzugt optische Nachweismethoden, welche beispielsweise die Messung von Absorption, Fluoreszenz, Circulardichroismus (CD), optische Rotationsdispersion (ORD), Refraktometrie, etc. umfassen, zur Anwendung. Besonders bevorzugt erfolgt der Nachweis des Analyten photometrisch oder fluorometrisch.

Zum Nachweis des Analyten kann ein Flüssigtest verwendet werden, wobei die erfindungsgemäßen Verbindungen beispielsweise in Form einer Lösung oder als Suspension in einer wässrigen oder nichtwässrigen Flüssigkeit vorliegen. Um die Löslichkeit insbesondere in wässrigen Flüssigkeiten zu erhöhen, können die erfindungsgemäßen polycyclischen Verbindungen oder

Vorläuferverbindungen desweiteren mindestens eine die Wasserlöslichkeit erhöhende funktionelle Gruppe, wie beispielsweise COOH oder SO₃H, umfassen, wobei sich letztere an einer beliebigen Position des Moleküls befinden kann. Es kann jedoch auch ein Trockentest verwendet werden, wobei die erfindungsgemäßen Verbindungen in diesem Fall beispielsweise auf einen geeigneten Träger, wie er oben beschrieben ist, aufgebracht sind.

Die Erfindung soll durch die nachfolgenden Figuren und Beispiele näher erläutert werden:

### Beschreibung der Figuren

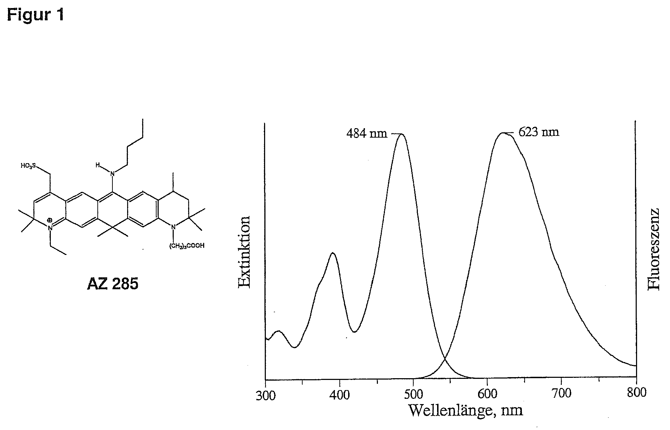

**Figur 1****:** Absorptions- und Fluoreszenzspektrum der polycylischen Verbindung 4 (AZ 285) in Ethanol.

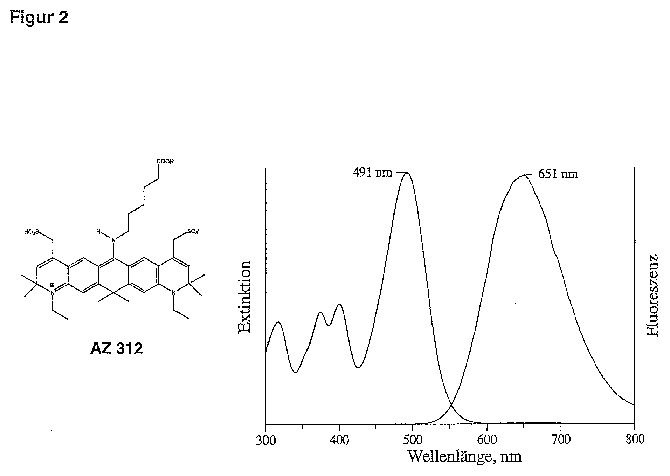

**Figur 2****:** Absorptions- und Fluoreszenzspektrum der polycylischen Verbindung 8 (AZ 312) in Ethanol.

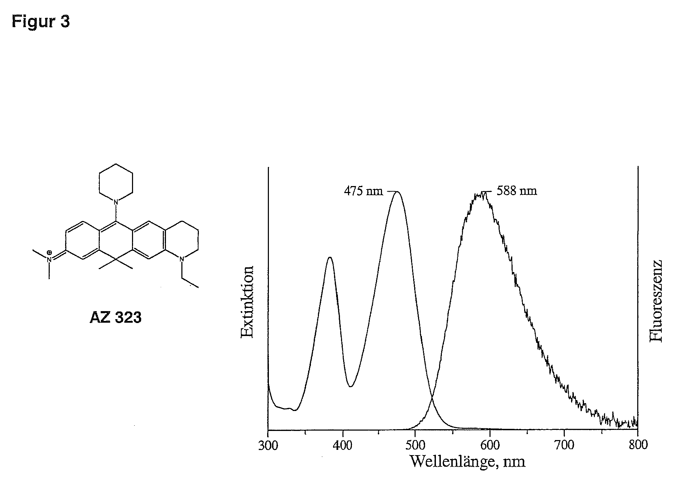

**Figur 3****:** Absorptions- und Fluoreszenzspektrum der polycylischen Verbindung 12 (AZ 323) in Ethanol.

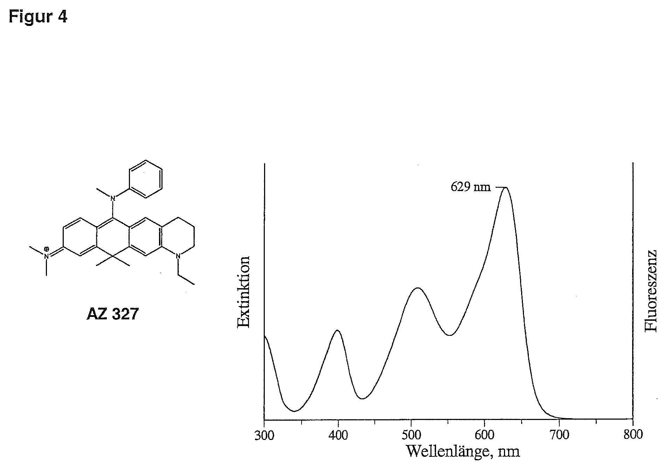

**Figur 4****:** Absorptionsspektrum der polycylischen Verbindung 14 (AZ 327) in Ethanol.

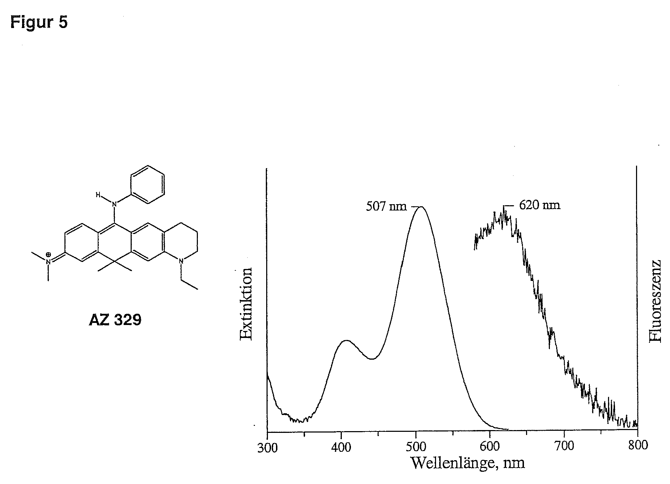

**Figur 5****:** Absorptions- und Fluoreszenzspektrum der polycylischen Verbindung 15 (AZ 329) in Ethanol.

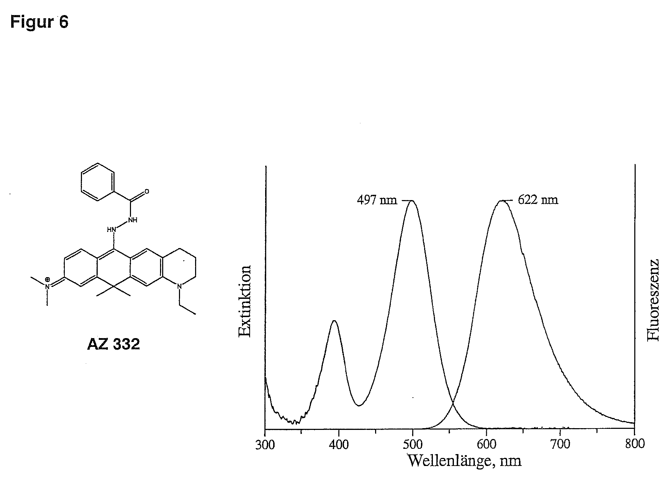

**Figur 6****:** Absorptions- und Fluoreszenzspektrum der polycylischen Verbindung 17 (AZ 332) in Ethanol.

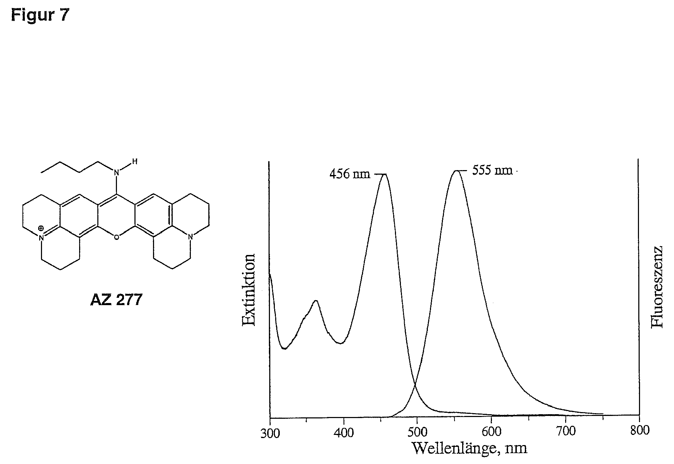

**Figur 7****:** Absorptions- und Fluoreszenzspektrum der polycylischen Verbindung 21 (AZ 277) in Ethanol (Vergleichsbeispiel).

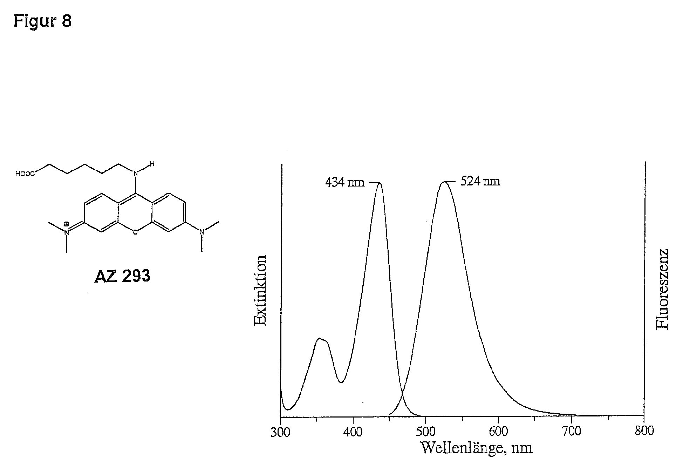

**Figur 8****:** Absorptions- und Fluoreszenzspektrum der polycylischen Verbindung 23 (AZ 293) in Ethanol (Vergleichsbeispiel).

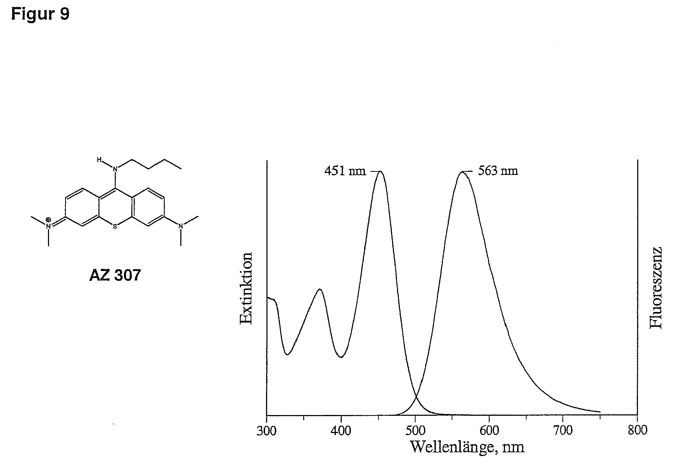

**Figur 9****:** Absorptions- und Fluoreszenzspektrum der polycylischen Verbindung 26 (AZ 307) in Ethanol (Vergleichsbeispiel).

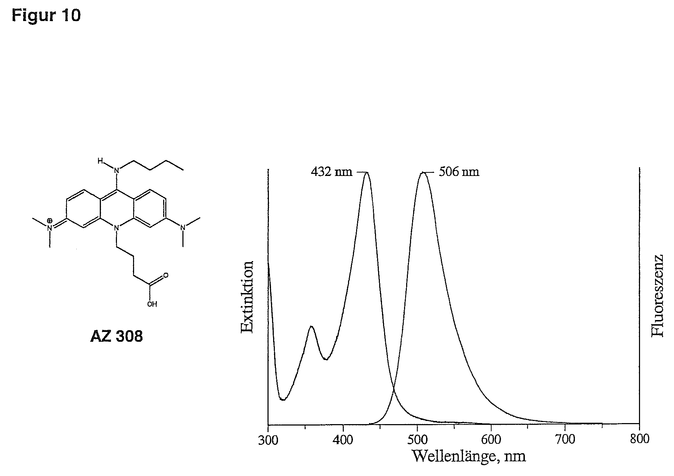

**Figur 10****:** Absorptions- und Fluoreszenzspektrum der polycylischen Verbindung 27 (AZ 308) in Ethanol (Vergleichsbeispiel).

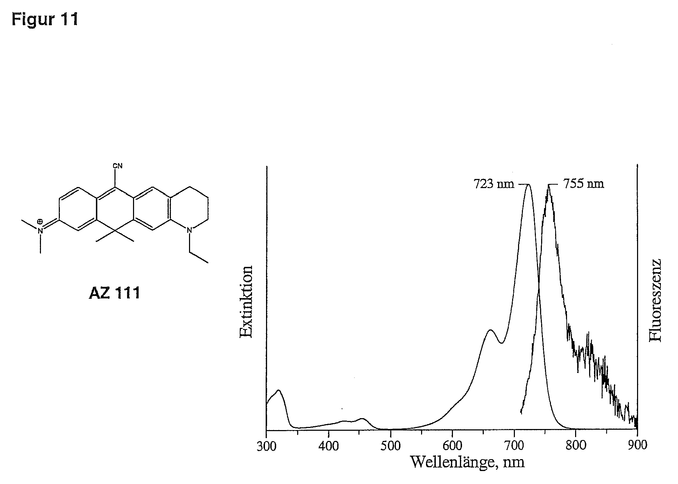

**Figur 11****:** Absorptions- und Fluoreszenzspektrum der polycylischen Verbindung 28 (AZ 111) in Ethanol.

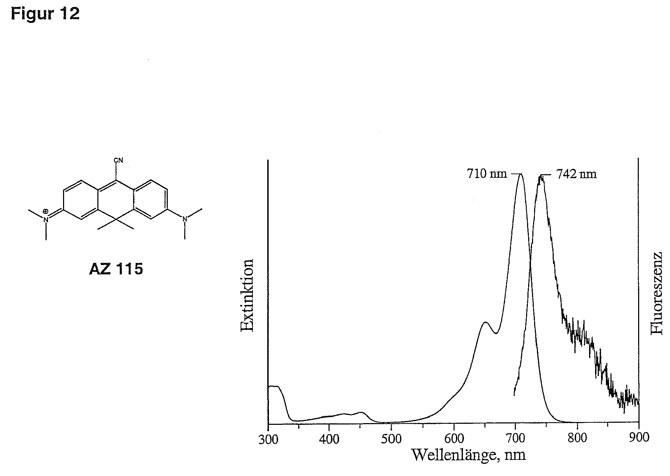

**Figur 12****:** Absorptions- und Fluoreszenzspektrum der polycylischen Verbindung 30 (AZ 115) in Ethanol.

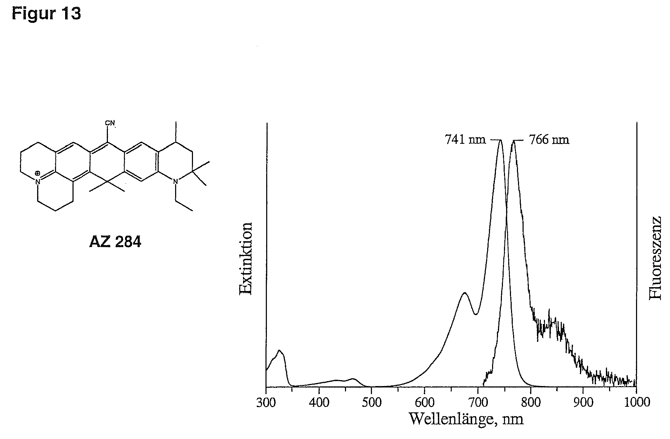

**Figur 13****:** Absorptions- und Fluoreszenzspektrum der polycylischen Verbindung 33 (AZ 284) in Ethanol.

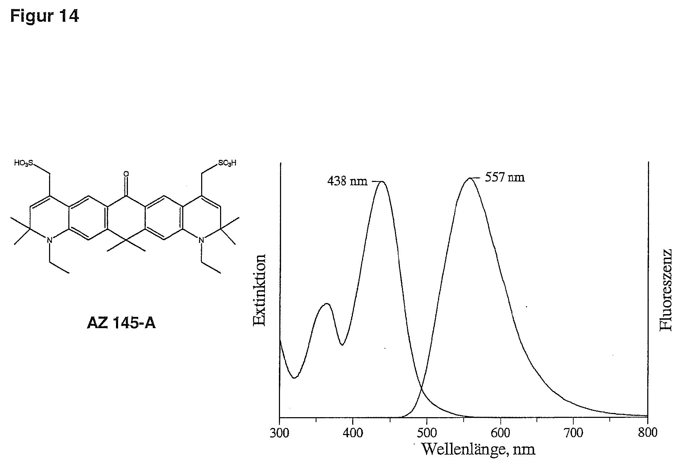

**Figur 14****:** Absorptions- und Fluoreszenzspektrum der polycylischen Verbindung 39 (AZ 145-A) in Ethanol.

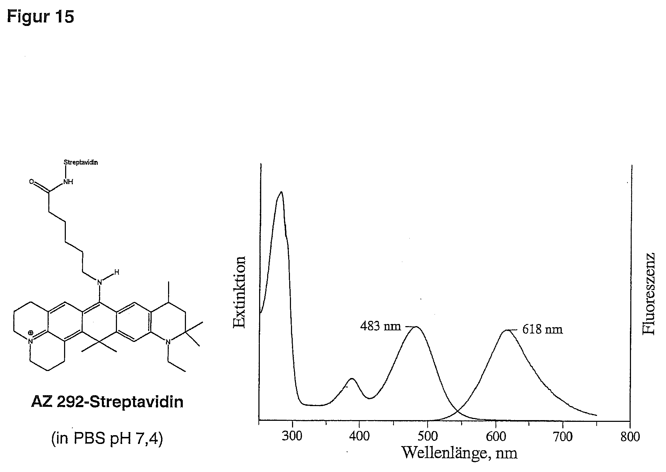

**Figur 15****:** Absorptions- und Fluoreszenzspektrum der polycylischen Verbindung 51 (AZ 292-Streptavidin) in PBS.

### Beispiele

### A. Herstellung polycyclischer Vorläuferverbindungen der allgemeinen Formel (III)

### Verbindung 40 (AZ 151-A)

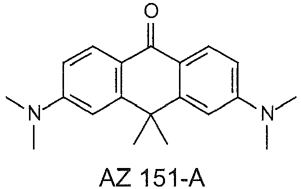

AZ 151-Perchlorat (2.44 g, 6.21 mmol) wird in 50 ml Aceton gelöst, portionsweise unter Eiskühlung mit fein pulverisiertem Kaliumpermanganat (3.5 g, 22.15 mmol) versetzt und anschließend 18 h verrührt. Man filtriert und wäscht den zurückbleibenden Braunstein mehrmals mit Chloroform und engt die vereinigten organischen Phasen bis zur Trockene ein.

Der Rückstand wird in Dichlormethan aufgenommen und an Kieselgel mit Dichlormethan/Methanol (100:0 - 99:1) säulenchromatographisch gereinigt. AZ 151-A (0.86 g, 2.79 mmol, 45%) wird als gelber kristalliner Feststoff erhalten (Schmelzbereich 195-200°C).

Optische Eigenschaften in Ethanol: λ_{abs} = 394 nm; λ_{fl} = 482 nm.

### B. Herstellung polycyclischer Vorläuferverbindungen der allgemeinen Formel (II)

### Verbindung 28 (AZ 111)

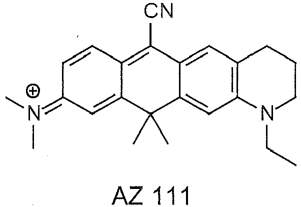

AZ 110-Perchlorat (250 mg, 0.58 mmol) wird in 25 ml trockenem Acetonitril gelöst und 1 h im N₂-Strom verrührt. Man versetzt mit 10 ml Tetrabutylammoniumcyanid in trockenem Acetonitril (310 mg, 1.16 mmol) und verrührt 2 h bei Raumtemperatur. Die zunächst intensiv blau gefärbte Lösung färbt sich schwach gelbgrün.

Zur Oxidation der Leukoverbindung versetzt man mit Chloranil (170 mg, 0.69 mmol) und verrührt unter Inertgas weitere 2 h bei Raumtemperatur. Die Reaktionsmischung färbt sich nach Zugabe des Oxidationsmittels spontan tiefgrün. Nach beendeter Reaktion wird bis zur Trockene eingeengt. Der Rückstand wird in Chloroform/Ethanol 98:2 aufgenommen und an Kieselgel mit einem von Chloroform nach Ethanol geführten Gradienten gereinigt, wobei der Farbstoff mit 5-15% Ethanol eluiert.

Die vereinigten Farbstofffraktionen werden bis zur Trockene eingeengt, in 10 ml Ethanol aufgenommen und in 150 ml 5%-ige Natriumperchloratlösung getropft. Der ausgefallene Farbstoff wird abgesaugt und im Vakuum über Phosphorpentoxid getrocknet. AZ 111 (114 mg, 0.32 mmol; 55%) wird als kristalliner grüner Feststoff erhalten.
LC-MS: 358.2
Optische Eigenschaften in Ethanol: λ_{abs} = 723 nm; λ_{fl} = 755 nm.

### Verbindung 29 (AZ 112)

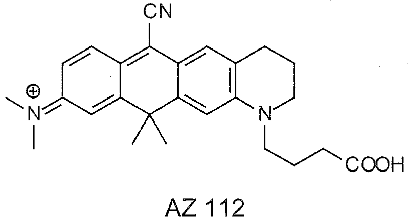

ATTO 610-Perchlorat (250 mg, 0.51 mmol), ein von der ATTO-TEC GmbH erhältlicher Farbstoff, wird in 25 ml trockenem Acetonitril gelöst und 1 h im N₂-Strom verrührt. Man versetzt mit 10 ml Tetrabutylammoniumcyanid (273 mg, 1.02 mmol) in trockenem Acetonitril und verrührt 2 h bei Raumtemperatur. Die zunächst intensiv blau gefärbte Lösung färbt sich schwach gelbgrün.

Zur Oxidation der Leukoverbindung versetzt man mit Chloranil (170 mg; 0.69 mmol) und verrührt unter Inertgas weitere 2 h bei Raumtemperatur. Die Reaktionsmischung färbt sich nach Zugabe des Oxidationsmittels spontan tiefgrün. Nach beendeter Reaktion wird bis zur Trockene eingeengt. Der Rückstand wird in Chloroform/Ethanol 95:5 aufgenommen und an Kieselgel mit einem von Chloroform nach Ethanol geführten Gradienten gereinigt, wobei der Farbstoff mit 10-20% Ethanol eluiert.

Die vereinigten Farbstofffraktionen werden bis zur Trockene eingeengt, in 10 ml Ethanol aufgenommen und in 150 ml 5%-ige Natriumperchloratlösung getropft. Der ausgefallene Farbstoff wird abgesaugt und im Vakuum über Phosphorpentoxid getrocknet. AZ 112 (116 mg, 0.29 mmol, 55%) wird als kristalliner grüner Feststoff erhalten.
LC-MS: 416.2
Optische Eigenschaften in Ethanol: λ_{abs} = 726 nm; λ_{fl} = 754 nm

### Verbindung 32 (AZ 147)

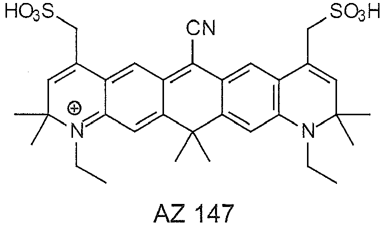

AZ 145-Natriumsalz (200 mg, 0.26 mmol) wird in 8 ml trockenem Dimethylformamid gelöst und 30 Minuten im N₂-Strom verrührt. Man versetzt mit 10 ml Tetrabutylammoniumcyanid (213 mg, 0.79 mmol) in trockenem Dimethylformamid und verrührt 2 h bei Raumtemperatur. Die zunächst intensiv blau gefärbte Lösung färbt sich schwach gelbgrün.

Zur Oxidation der Leukoverbindung versetzt man mit Chloranil (78 mg, 0.32 mmol) und verrührt unter Inertgas weitere 2 h bei Raumtemperatur. Die Reaktionsmischung färbt sich nach Zugabe des Oxidationsmittels allmählich tiefgrün. Nach beendeter Reaktion wird bis zur Trockene eingeengt. Der Rückstand wird in Chloroform/Ethanol 85:15 aufgenommen und an Kieselgel mit einem von Chloroform nach Ethanol geführten Gradienten gereinigt, wobei der Farbstoff mit 40-50 % Ethanol eluiert.

Die vereinigten Farbstofffraktionen werden bis zur Trockene eingeengt. Man nimmt in wenig Ethanol auf und tropft in 100 ml Diethylether. Der ausgefallene Farbstoff wird abgesaugt und im Vakuum über Phosphorpentoxid getrocknet. AZ 147 (110 mg, 0.17 mmol, 65%) wird als kristalliner grüner Feststoff erhalten.
LC-MS: 638.3
Optische Eigenschaften in Ethanol: λ_{abs} = 801 nm, λ_{fl} = 830 nm

### Verbindung 33 (AZ 284)

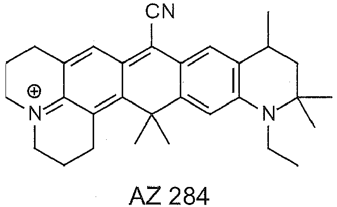

AZ 124-Perchlorat (250 mg, 0.47 mmol) wird in 25 ml trockenem Acetonitril gelöst und 1 h im N₂-Strom verrührt. Man versetzt mit 10 ml Tetrabutylammoniumcyanid (255 mg, 0.95 mmol) in trockenem Acetonitril und verrührt 2 h bei Raumtemperatur. Die zunächst intensiv blau gefärbte Lösung färbt sich schwach gelbgrün.

Zur Oxidation der Leukoverbindung versetzt man mit Chloranil (140 mg, 0.57 mmol) und verrührt unter Inertgas weitere 2 h bei Raumtemperatur. Die Reaktionsmischung färbt sich nach Zugabe des Oxidationsmittels spontan tief grün. Nach beendeter Reaktion wird bis zur Trockene eingeengt. Der Rückstand wird in Chloroform/Ethanol 98:2 aufgenommen und an Kieselgel mit einem von Chloroform nach Ethanol geführten Gradienten gereinigt, wobei der Farbstoff mit 5-10% Ethanol eluiert.

Die vereinigten Farbstofffraktionen werden bis zur Trockene eingeengt, in 10 ml Ethanol aufgenommen und in 150 ml 5%-ige Natriumperchloratlösung getropft. Der ausgefallene Farbstoff wird abgesaugt und im Vakuum über Phosphorpentoxid getrocknet. AZ 284 (137 mg, 0.30 mmol, 64%) wird als kristalliner grüner Feststoff erhalten.
LC-MS: 452.3
Optische Eigenschaften in Ethanol: λ_{abs} = 741 nm; λ_{fl} = 766 nm

### C. Herstellung polycyclischer Verbindungen der allgemeinen Formel (I)

### Verbindung 5 (AZ 286)

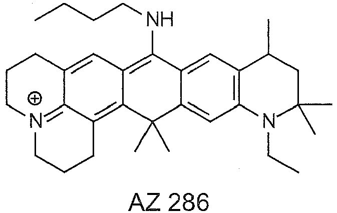

AZ 284-Perchlorat (250 mg, 0.45 mmol) wird in 40 ml Acetonitril gelöst und mit n-Butylamin (166 mg, 2.26 mmol) versetzt. Die Reaktionsmischung wird 4 h bei Raumtemperatur verrührt, wobei sich die Lösung gelborange färbt und eine intensive grüne Fluoreszenz zeigt.

Die Reaktionslösung wird bis zur Trockene eingeengt, in 15 ml Wasser/Aceton/TFA im Verhältnis 700/300/0.1 aufgenommen, filtriert (0.45 µm) und an Kieselgel RP 18 mit einem von Wasser nach Aceton geführten Gradienten gereinigt.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 286 (238 mg, 0.40 mmol, 88%) wird als orangefarbener kristalliner Feststoff erhalten.
LC-MS: 338.3
Optische Eigenschaften in Ethanol: λ_{abs} = 480 nm, λ_{fl} = 601 nm, η_{fl} = 79%

### Verbindung 7 (AZ 292)

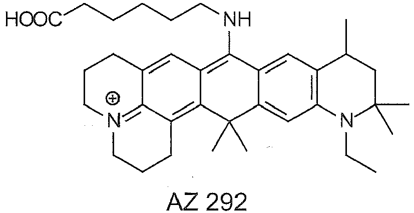

AZ 284-Perchlorat (190 mg, 0.34 mmol) wird in 6 ml DMSO gelöst und mit 350 µl einer 2M Lösung von 6-Aminohexansäure Kaliumsalz (203 mg, 1.20 mmol) in Wasser versetzt. Die Reaktionslösung färbt sich spontan von grün nach tief braunrot.

Es wird 1 h bei Raumtemperatur verrührt. Die Reaktionslösung wird mit 17 ml Wasser/Aceton/TFA im Verhältnis 800/200/0.1 versetzt, filtriert (0.45 µm) und an Kieselgel RP 18 mit einem von Wasser nach Aceton geführten Gradienten gereinigt.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 292 (165 mg, 0.25 mmol, 77%) wird als orangefarbener kristalliner Feststoff erhalten.
LC-MS: 556.4
Optische Eigenschaften in Ethanol: λ_{abs} = 480 nm; λ_{fl} = 601 nm, η_{fl} = 78%

### Verbindung 8 (AZ 312)

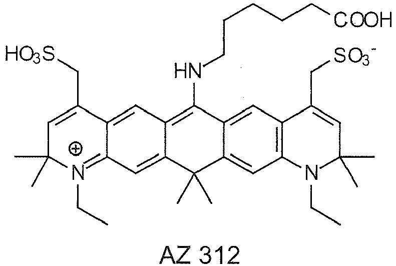

AZ 147-Natriumsalz (110 mg, 0.638 mmol) wird in 5 ml DMSO gelöst und mit 200 µl einer 2M Lösung von 6-Aminohexansäure Kaliumsalz (68 mg, 0.40 mmol) in Wasser versetzt. Die Reaktionslösung färbt sich spontan von grüngelb nach tief braunrot.

Es wird 1 h bei Raumtemperatur verrührt. Man versetzt mit 18 ml Wasser/Aceton/TFA im Verhältnis 950:50:0.1 und filtriert (0.45 µm). Das Filtrat wird an Kieselgel RP18 mit einem von Wasser nach Aceton geführten Gradienten gereinigt, wobei das Produkt mit Wasser/Aceton/TFA im Verhältnis 700:300:0.1 eluiert.

Die Produktfraktionen werden bis zur Trockene eingeengt. AZ 312 (88 mg, 0.12 mmol, 69%) wird als orangeroter kristalliner Feststoff erhalten.
LC-MS: 742.3
Optische Eigenschaften in Ethanol: λ_{abs} = 491 nm; λ_{fl} = 651 nm, η_{fl} = 61%
Optische Eigenschaften in PBS-Puffer pH 7.4:
λ_{abs} = 490 nm; λ_{fl} = 670 nm, η_{fl} = 26%

### Verbindung 14 (AZ 327)

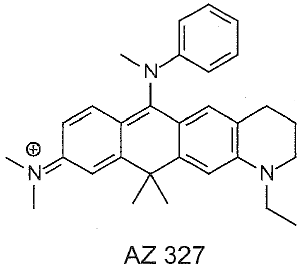

AZ110-A (60 mg, 0.17 mmol) wird in 4 ml Dichlormethan gelöst und tropfenweise mit Trifluormethansulfonsäureanhydrid (145 µl, 0.86 mmol) versetzt. Die zunächst tiefblau, später blaugrün gefärbte Lösung wird 10 Minuten bei 25°C verrührt. Man versetzt tropfenweise mit N-Methylanilin (186 mg, 1.72 mmol) und rührt 3 h bei 25°C.

Die Reaktionslösung wird bis zur Trockene eingeengt, der Rückstand in 12 ml Wasser/Aceton/TFA im Verhältnis 600:400:0.1 aufgenommen, filtriert (0.45 µm) und an Kieselgel RP18 mit einem von Wasser nach Aceton geführten Gradienten gereinigt, wobei der Farbstoff mit 400:600:0.1 eluiert.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 327 (79 mg, 0.15 mmol, 85%) wird als blauer kristalliner Feststoff erhalten.
LC-MS: 438.2
Optische Eigenschaften in Acetonitril: λ_{abs} = 629 nm, η_{fl} = < 0.1%

### Verbindung 15 (AZ 329)

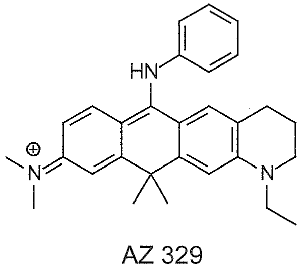

AZ110-A (60 mg, 0.17 mmol) wird in 4 ml Dichlormethan gelöst und tropfenweise mit Trifluormethansulfonsäureanhydrid (145 µl, 0.86 mmol) versetzt. Die zunächst tiefblau, später blaugrün gefärbte Lösung wird 10 Minuten bei 25°C verrührt. Man versetzt tropfenweise mit Anilin (157 µl, 1.72 mmol) und rührt 30 Minuten bei 25°C.

Die Reaktionslösung wird bis zur Trockene eingeengt, der Rückstand in 12 ml Wasser/Aceton/TFA im Verhältnis 700:300:0.1 aufgenommen, filtriert (0.45 µm) und an Kieselgel RP18 mit einem von Wasser nach Aceton geführten Gradienten gereinigt, wobei der Farbstoff mit 500:500:0.1 eluiert.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 329 (61 mg, 0.12 mmol, 68%) wird als dunkelroter kristalliner Feststoff erhalten.
LC-MS: 424.2
Optische Eigenschaften in Ethanol: λ_{abs} = 507 nm, λ_{fl} = 620 nm, η_{fl} < 1%

### Verbindung 21 (AZ 277) (Vergleichsbeispiel)

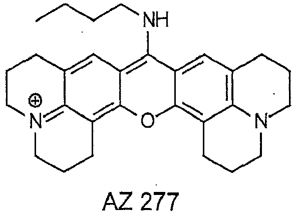

Rhodamin 800-Perchlorat (60 mg, 0.12 mmol) wird in 15 ml Acetonitril gelöst und mit n-Butylamin (44 mg, 0.61 mmol) versetzt. Die Reaktionsmischung wird 2 h bei Raumtemperatur verrührt, wobei sich die Lösung gelborange färbt und eine intensive grüne Fluoreszenz zeigt.

Die Reaktionslösung wird bis zur Trockene eingeengt, in 15 ml Wasser/Aceton/TFA im Verhältnis 600/400/0.1 aufgenommen, filtriert (0.45 µm) und an Kieselgel RP 18 mit einem von Wasser nach Aceton geführten Gradienten gereinigt.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 277 (54 mg, 0.10 mmol, 82%) wird als gelber kristalliner Feststoff erhalten.
LC-MS: 442.3
Optische Eigenschaften in Ethanol: λ_{abs} = 456 nm; λ_{fl} = 555 nm, η_{fl} = 82%

### Verbindung 22 (AZ 278) (Vergleichsbeispiel)

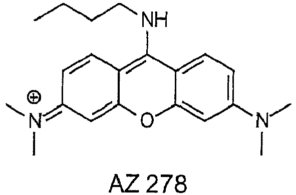

9-Cyanotetramethylpyronin-Perchlorat (250 mg, 0.64 mmol) wird in 40 ml Acetonitril gelöst und mit n-Butylamin (233 mg, 3.19 mmol) versetzt. Die Reaktionsmischung wird 2 h bei Raumtemperatur verrührt, wobei sich die Lösung gelborange färbt und eine intensive grüne Fluoreszenz zeigt.

Die Reaktionslösung wird bis zur Trockene eingeengt, in 15 ml Wasser/Aceton/TFA im Verhältnis 700/300/0.1 aufgenommen, filtriert (0.45 µm) und an Kieselgel RP 18 mit einem von Wasser nach Aceton geführten Gradienten gereinigt.

Die Produktfraktionen werden vereinigt, mit 5 ml 10%-iger Natriumperchloratlösung versetzt und am Rotationsverdampfer eingeengt. Der dabei ausfallende Farbstoff wird abgesaugt und im Vakuum über Phosphorpentoxid getrocknet. AZ 278 (225 mg, 0.51 mmol, 81%) wird als gelber kristalliner Feststoff erhalten.
LC-MS: 338.3
Optische Eigenschaften in Ethanol: λ_{abs} = 433 nm; λ_{fl} = 524 nm, η_{fl} = 47%

## Patentansprüche

1. Polycyclische Verbindung der allgemeinen Formel (I) 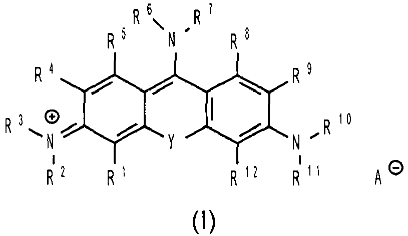 worin
R¹, R⁴, R⁵, R⁸, R⁹ und R¹² unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R², R³, R¹⁰ und R¹¹ unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
R⁶ und R⁷ unabhängig voneinander Wasserstoff, NR¹³R¹⁴, eine Kohlenwasserstoffgruppe mit 1-20 C-Atomen, oder eine Analytmolekülgruppe bedeutet, wobei R¹³ und R¹⁴ unabhängig voneinander Wasserstoff, Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H oder eine Kohlenwasserstoffgruppe mit 1-20 C-Atomen bedeutet, und wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
oder R⁶ und R⁷ zusammen mit dem N-Atom, an welches sie gebunden sind, einen 3- bis 7-gliedrigen Ring bilden, wobei der Ring eine oder mehrere Doppelbindungen oder/und ein oder mehrere zusätzliche Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst,
Y CR¹⁵R¹⁶ bedeutet, wobei R¹⁵ und R¹⁶ unabhängig voneinander Wasserstoff, CN, COOH, COO(Alkyl), COO(Aryl), PO₃H₂, SO₃H oder eine Kohlenwasserstoffgruppe mit 1-20 C-Atomen bedeutet, und wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst, oder
R¹⁵ und R¹⁶ zusammen mit dem C-Atom, an welches sie gebunden sind, einen 3- bis 7-gliedrigen Ring bilden, wobei der Ring eine oder mehrere Doppelbindungen oder/und ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfassen kann oder/und mit einem oder mehreren 3- bis 7-gliedrigen Ringen anelliert sein kann, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst, und
A⁻ ein Anion bedeutet,
oder worin R¹ und R², R³ und R⁴, R⁹ und R¹⁰ oder/und R¹¹ und R¹² zusammen mit den Atomen, an welche sie gebunden sind, einen 5- oder 6-gliedrigen Ring bilden, wobei der Ring gegebenenfalls eine oder mehrere Doppelbindungen oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂, SO₃H und einer Kohlenwasserstoffgruppe mit 1-20 C-Atomen umfasst, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, CN, OH, O(Alkyl), O(Aryl), SH, S(Alkyl), S(Aryl), NH₂, NH(Alkyl), NH(Aryl), N(Alkyl)₂, N(Aryl)₂, NO₂, CHO, COOH, COO(Alkyl), COO(Aryl), PO₃H₂ und SO₃H umfasst.

2. Polycyclische Verbindung nach Anspruch 1,
**dadurch gekennzeichnet,**
**dass** R¹ und R², R³ und R⁴, R⁹ und R¹⁰ oder/und R¹¹ und R¹² zusammen mit den Atomen, an welche sie gebunden sind, einen 5 oder 6-gliedrigen Ring bilden.

3. Polycyclische Verbindung nach Anspruch 2,
**dadurch gekennzeichnet,**
**dass** mindestens ein 5- oder 6-gliedriger Ring mit einer oder mehreren Kohlenwasserstoffgruppen mit 1-20 C-Atomen substituiert ist, wobei die Kohlenwasserstoffgruppe(n) gegebenenfalls einen oder mehrere Substituenten ausgewählt aus der Gruppe bestehend aus COOH, COO(Alkyl), COO(Aryl) und SO₃H umfasst/umfassen.

4. Polycyclische Verbindung nach einem der Ansprüche 1 bis 3,
**dadurch gekennzeichnet,**
**dass** R², R³, R¹⁰ oder/und R¹¹ eine Kohlenwasserstoffgruppe mit 1-20 C-Atomen, insbesondere eine Alkylgruppe mit 1-6 C-Atomen, bedeutet, oder/und mindestens zwei Reste ausgewählt aus der Gruppe bestehend aus R⁵, R⁸ und R¹², insbesondere R⁵ und R⁸, Wasserstoff bedeuten.

5. Polycyclische Verbindung nach einem der Ansprüche 1 bis 4,
**dadurch gekennzeichnet,**
**dass** R⁶ und R⁷ unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome ausgewählt aus der Gruppe bestehend aus N, O und S oder/und einen oder mehrere Substituenten ausgewählt aus der Gruppe bestehend aus Halogen, OH, SH, NH₂, COOH, COO(Alkyl), COO(Aryl) und SO₃H umfasst.

6. Polycyclische Verbindung nach einem der Ansprüche 1 bis 5,
**dadurch gekennzeichnet,**
**dass** R⁶ und R⁷ nicht gleichzeitig Wasserstoff bedeuten.

7. Polycyclische Verbindung nach einem der Ansprüche 1 bis 6,
**dadurch gekennzeichnet,**
**dass** Y CR¹⁵R¹⁶ bedeutet, wobei R¹⁵ und R¹⁶ unabhängig voneinander eine Kohlenwasserstoffgruppe mit 1-20 C-Atomen, insbesondere eine Alkylgruppe mit 1-6 C-Atomen, bedeutet.

8. Verfahren zur Herstellung einer polycyclischen Verbindung der allgemeinen Formel (I),
**dadurch gekennzeichnet,**
**dass** eine polycyclische Vorläuferverbindung der allgemeinen Formel (II) 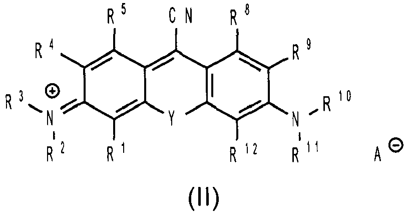 oder eine polycyclische Vorläuferverbindung der allgemeinen Formel (III) 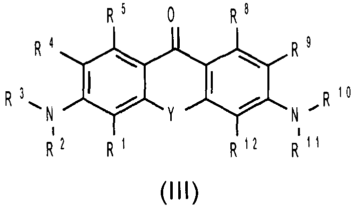 mit einem primären oder sekundären Amin der allgemeinen Formel HNR⁶R⁷ umgesetzt wird,
wobei R¹, R², R³, R⁴, R⁵, R⁶, R⁷, R⁸, R⁹, R¹⁰, R¹¹, R¹², Y und A⁻ wie in einem der Ansprüche 1 bis 7 definiert sind.

9. Verfahren nach Anspruch 8,
**dadurch gekennzeichnet,**
**dass** die Umsetzung bei einer Temperatur von mindestens 0°C, insbesondere bei einer Temperatur im Bereich von 15°C bis 35°C, oder/und in Gegenwart eines Lösungsmittels, insbesondere eines Lösungsmittels ausgewählt aus der Gruppe bestehend aus Aceton, Acetonitril, Chloroform, Dichlormethan, Dimethylsulfoxid, N,N-Dimethylformamid und Gemischen hiervon, durchgeführt wird.

10. Polycylische Vorläuferverbindung der allgemeinen Formel (III) 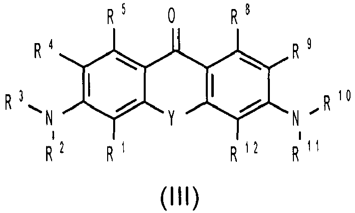
worin R¹, R², R³, R⁴, R⁵, R⁸, R⁹, R¹⁰, R¹¹ und R¹² wie in einem der Ansprüche 1 bis 7 definiert sind und
Y CR¹⁵R¹⁶ bedeutet, wobei R¹⁵ und R¹⁶ wie in Anspruch 1 definiert sind.

11. Verfahren zur Herstellung einer polycylischen Vorläuferverbindung der allgemeinen Formel (III),
**dadurch gekennzeichnet,**
**dass** eine polycyclische Vorläuferverbindung der allgemeinen Formel (IV) 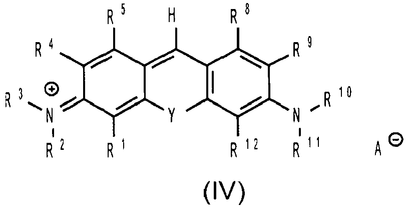 mit einem Oxidationsmittel umgesetzt wird,
wobei R¹, R², R³, R⁴, R⁵, R⁸, R⁹, R¹⁰, R¹¹, R¹², Y und A⁻ wie in einem der Ansprüche 1 bis 7 definiert sind.

12. Verwendung einer polycyclischen Verbindung nach einem der Ansprüche 1 bis 7 oder einer polycyclischen Vorläuferverbindung nach Anspruch 10 zur qualitativen oder/und quantitativen Bestimmung eines Analyten in einer Probe.

13. Verwendung nach Anspruch 12,
**dadurch gekennzeichnet,**
**dass** die Bestimmung eine Markierung des Analyten durch kovalente Bindung der polycyclischen Verbindung oder der polycyclischen Vorläuferverbindung an den Analyten umfasst oder/und es sich bei dem Analyten um ein Biomolekül, insbesondere um ein Peptid, ein Polypeptid, ein Protein, ein Nukleotid, ein Polynukleotid, ein Nukleosid, eine Nukleinsäure, ein Nukleinsäureanalogon oder ein Hapten, handelt.

## Claims

1. Polycyclic compound with the general formula (I) 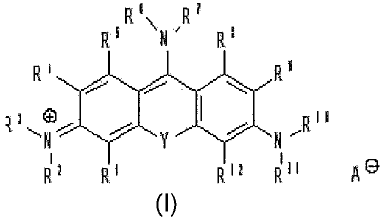 where
R¹, R⁴, R⁵, R⁸, R⁹ and R¹² independently stand for hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H or a hydrocarbon group with 1-20 C-atoms, wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
where R², R³, R¹⁰ and R¹¹ independently stand for hydrogen or a hydrocarbon group with 1-20 C-atoms, wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
where R⁶ and R⁷ independently stand for hydrogen, NR¹³R¹⁴, a hydrocarbon group with 1-20 C-atoms or an analyte molecule group, wherein R13 and R14 independently comprise hydrogen, halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H or a hydrocarbon group with 1-20 C-atoms, and wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
or where R⁶ and R⁷ together with the N-atom, to which they are bound, form a 3 to 7-unit ring, wherein the ring can comprise one or more double bonds or/and one or more additional heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H and a hydrocarbon group with 1-20 C-atoms or/and can be in annulation with one or more 3 to 7-unit rings, wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H,
where Y stands for CR¹⁵R¹⁶, wherein R¹⁵ and R¹⁶ independently stand for hydrogen, CN, COOH, COO(alkyl), COO(aryl), PO₃H₂, SO₃H or a hydrocarbon group with 1-20 C-atoms, and wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H, or
where R¹⁵ and R¹⁶ together with the C-atom, to which they are bound form a 3-to 7-unit ring, wherein the ring can comprise one or more double bonds or/and one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H and a hydrocarbon group with 1-20 C-atoms or/and can be in annulation with one or more 3-unit to 7-unit rings, wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H, and
where A⁻ stands for an anion,
or where R¹and R², R³ and R⁴, R⁹ and R¹⁰ or/and R¹¹ and R¹² together with the atoms to which they are bound form a 5- or 6-unit ring, wherein the ring can comprise, if applicable, one or more double bonds or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂, SO₃H and a hydrocarbon group with 1-20 C-atoms wherein the hydrocarbon group comprises, if applicable, one or more heteroatoms, selected from the group consisting of N, O and S or/and one or more substituents, selected from the group consisting of halogen, CN, OH, O(alkyl), O(aryl), SH, S(alkyl), S(aryl), NH₂, NH(alkyl), NH(aryl), N(alkyl)₂, N(aryl)₂, NO₂, CHO, COOH, COO(alkyl), COO(aryl), PO₃H₂ and SO₃H.

2. Polycyclic compound according to claim 1,
**characterized in that**
R¹and R2, R³ and R⁴, R⁹ and R¹⁰ or/and R¹¹ and R¹² form a 5- or 6-unit ring together with the atoms to which they are bound.

3. Polycyclic compound according to claim 2,
**characterized in that**
at least one 5- or 6-unit ring is substituted with one or more hydrocarbon groups with 1-20 C-atoms, wherein the hydrocarbon group(s) comprise, if applicable, one or more substituents selected from the group consisting of COOH, COO(alkyl), COO(aryl) and SO₃H.

4. Polycyclic compound according to one of claims 1 to 3,
**characterized in that**
R², R³, R¹⁰ or/and R¹¹ stand for a hydrocarbon group with 1-20 C-atoms, in particular an alkyl group with 1-6 C-atoms, or/and **in that** at least two groups selected from the group comprising R⁵, R⁸and R¹², in particular R⁵ and R⁶ stand for hydrogen.

5. Polycyclic compound according to one of claims 1 to 4,
**characterized in that**
R⁶ and R⁷ independently stand for hydrogen or a hydrocarbon group with 1-20 C-atoms, wherein the hydrocarbon group comprises, if applicable, one or more heteroatoms, selected from the group consisting of N, O and S or/and one or more substituents, selected from the group consisting of halogen, OH, SH, NH₂, COOH, COO(alkyl), COO(aryl) and SO₃H.

6. Polycyclic compound according to one of claims 1 to 5,
**characterized in that**
R⁶ and R⁷ do not simultaneously stand for hydrogen.

7. Polycyclic compound according to one of claims 1 to 6,
**characterized in that**
_{Υ} stands for CR¹⁵R¹⁶, wherein R¹⁵ and R¹⁶ independently stand for a hydrocarbon group with 1-20 C-atoms, in particular an alkyl group with 1-6 C-atoms.

8. Method for producing a polycyclic compound with the general formula (I),
**characterized in that**
a polycyclic precursor with the general formula (II) 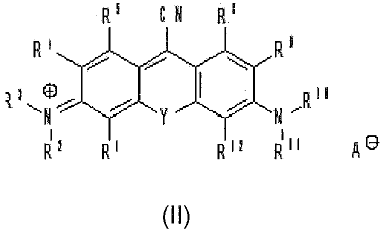 or a polycyclic precursor with the general formula (III) 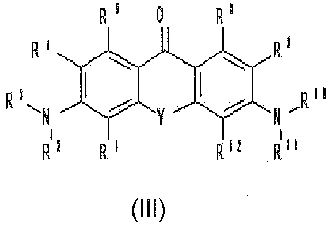 is converted with a primary or a secondary amine with the general formula HNR⁶R⁷,
wherein R¹, R², R³, R⁴, R⁵, R⁶, R⁷, R⁸, R⁹, R¹⁰, R¹¹, R¹², Y and A⁻ are defined according to one of claims 1 to 7.

9. Method according to claim 8,
**characterized in that**
the conversion is carried out at a temperature of at least 0°C, in particular at a temperature in the range of 15°C to 35°C or/and in the presence of a solvent, in particular a solvent selected from the group consisting of acetone, acetonitrile, chloroform, dichloromethane, dimethyl sulfoxide, N,N dimethyl formamide and mixtures thereof.

10. Polycyclic precursor with the general formula (III) 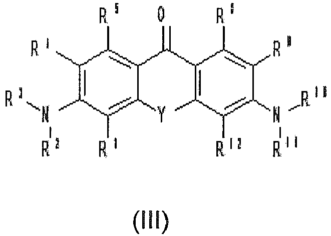 wherein R¹, R², R³, R⁴, R⁵, R⁸, R⁹, R¹⁰, R¹¹ and R¹² are defined according to one of claims 1 to 7 and wherein Y stands for CR¹⁵R¹⁶, R¹⁵ and wherein R¹⁶ is defined according to claim 1.

11. Method for producing a polycyclic precursor with the general formula (III),
**characterized in that**
a polycyclic precursor with the general formula (IV) 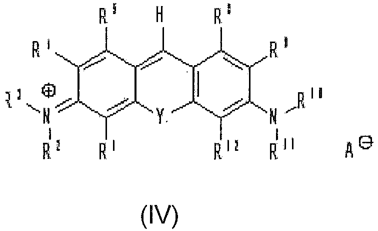 is converted with an oxidizing agent,
wherein R¹, R², R³, R⁴, R⁵, R⁸, R⁹, R¹⁰, R¹¹, R¹², Y and A⁻ are defined according to one of claims 1 to 7.

12. Application of a polycyclic compound according to one of claims 1 to 7 or a polycyclic precursor according to claim 10 for the qualitative or/and quantitative determination of an analyte in a sample.

13. Application according to claim 12,
**characterized in that**
the determination comprises labelling the analyte by covalent binding of the polycyclic compound or the polycyclic precursor to the analyte or/and **in that** the analyte is a biomolecule, in particular a peptide, a polypeptide, a proteine, a nucleotide, a polynucleotide, a nucleoside, a nucleic acid, a nucleic acid analogue or a hapten.

## Revendications

1. Composé polycyclique avec la formule générale (I) 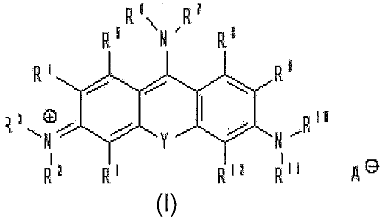 où
R¹, R⁴, R⁵, R⁸, R⁹ et R¹² représentent indépendamment hydrogène, halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H ou un groupe hydrocarbure avec 1-20 atomes C, où le groupe hydrocarbure comprend, si applicable, un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
où R², R³, R¹⁰ et R¹¹ représentent indépendamment hydrogène ou un groupe hydrocarbure avec 1-20 atomes C, où le groupe hydrocarbure comprend, si applicable, un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
où R⁶ et R⁷ représentent indépendamment hydrogène, NR¹³R¹⁴, un groupe hydrocarbure avec 1-20 atomes C ou un groupe de molécules d'analytes, où
R¹³ et R¹⁴ représentent indépendamment hydrogène, halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H ou un groupe hydrocarbure avec 1-20 atomes C, où le groupe hydrocarbure comprend, si applicable, un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
ou, où R⁶ et R⁷ avec l'atome N, auquel ils sont liés, forment un anneau à 3-7 éléments, où l'anneau peut comprendre une ou plusieurs double liaisons ou/et un ou plusieurs hétéroatomes supplémentaires, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H et un groupe hydrocarbure avec 1-20 atomes C ou/et où l'anneau peut être condensé avec un ou plusieurs anneaux à 3-7 éléments, où le groupe hydrocarbure peut, si applicable, comprendre un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H,
où Y signifie CR¹⁵R¹⁶, R¹⁵ et R¹⁶ signifiant indépendamment hydrogène, CN, COOH, COO(alkyle), COO(aryle), PO₃H₂, SO₃H ou un groupe hydrocarbure avec 1-20 atomes C et où le groupe hydrocarbure comprend, si applicable, un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃, ou
où R¹⁵ et R¹⁶ forment avec l'atome C auquel ils sont liés, un anneau à 3-7 éléments, où l'anneau peut comprendre une ou plusieurs double liaisons ou/et un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H et un groupe hydrocarbure avec 1-20 atomes C ou/et où l'anneau peut être condensé avec un ou plusieurs anneaux à 3-7 éléments, où le groupe hydrocarbure peut, si applicable, comprendre un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H, et
où A⁻ représente un anion,
ou, où R¹ et R², R³ et R⁴, R⁹ et R¹⁰ ou/et R¹¹ et R¹² forment avec les atomes auxquels ils sont liés, un anneau à 5 ou 6 éléments, où l'anneau peut comprendre une ou plusieurs double liaisons ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂, SO₃H, et un groupe hydrocarbure avec 1-20 atomes C où le groupe hydrocarbure peut, si applicable, comprendre un ou plusieurs hétéroatomes, sélectés du groupe comprenant N, O et S ou/et un ou plusieurs substituants, sélectés du groupe comprenant de l'halogène, CN, OH, O(alkyle), O(aryle), SH, S(alkyle), S(aryle), NH₂, NH(alkyle), NH(aryle), N(alkyle)₂, N(aryle)₂, NO₂, CHO, COOH, COO(alkyle), COO(aryle), PO₃H₂ et SO₃H.

2. Composé polycyclique selon la revendication 1,
**caractérisé en ce que**
R¹ et R², R³ et R⁴, R⁹ et R¹⁰ ou/et R¹¹ et R¹² forment avec les atomes auxquels ils sont liés un anneau à 5 ou 6 éléments.

3. Composé polycyclique selon la revendication 2,
**caractérisé en ce que**
au moins un anneau à 5 ou 6 éléments est substitué par un ou plusieurs groupes hydrocarbure avec 1-20 atomes C, le(s) groupe(s) hydrocarbure comprenant, si applicable, un ou plusieurs substituants sélectés du groupe comprenant COOH, COO(alkyle), COO(aryle) et SO₃H.

4. Composé polycyclique selon la revendication 1 à 3,
**caractérisé en ce que**
R², R³, R¹⁰ ou/et R¹¹ représentent un groupe hydrocarbure avec 1-20 atomes C, en particulier un groupe alkyle avec 1-6 atomes, ou/et **en ce qu'**au moins deux groupes sélectés du groupe comprenant R⁵, R⁸ et R¹², en particulier R⁵ et R⁶ représentent hydrogène.

5. Composé polycyclique selon la revendication 1 à 4,
**caractérisé en ce que**
R⁶ et R⁷ représentent indépendamment hydrogène ou un groupe hydrocarbure avec 1-20 atomes C, le groupe hydrocarbure comprenant, si applicable, un ou plusieurs hétéroatomes sélectés du groupe comprenant N, O et S ou/et un ou plusieurs substituants, sélectés du groupe comprenant de l'halogène, OH, SH, S(alkyle), NH₂, COOH, COO(alkyle), COO(aryle) et SO₃H.

6. Composé polycyclique selon la revendication 1 à 5,
**caractérisé en ce que**
R⁶ et R⁷ ne représentent pas simultanément hydrogène.

7. Composé polycyclique selon la revendication 1 à 6,
**caractérisé en ce que**
Y représente CR¹⁵R¹⁶, où R¹⁵ et R¹⁶ représentent indépendamment un groupe hydrocarbure avec 1-20 atomes, en particulier un groupe alkyle avec 1-6 atomes.

8. Méthode pour produire un composé polycyclique avec la formule générale (I),
**caractérisé en ce que**
un précurseur polycyclique avec la formule générale (II) 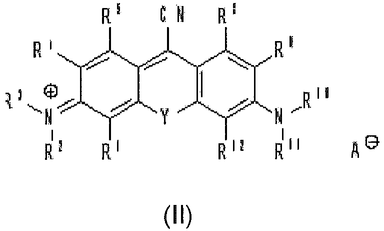 ou un précurseur polycyclique avec la formule générale (III) 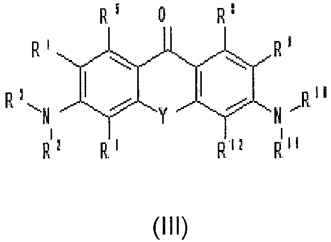 est transformé avec une amine primaire ou secondaire de la formule générale HNR⁶R⁷,
où R¹, R², R³, R⁴, R⁵, R⁶, R⁷, R⁸, R⁹, R¹⁰, R¹¹, R¹², Y et A⁻ sont définis selon une des revendications 1 à 7.

9. Méthode selon la revendication 8,
**caractérisé en ce que**
la transformation est réalisée à une température d'au moins 0°C, en particulier à une température dans la gamme comprise entre 15°C et 35°C ou/et en présence d'un solvant, en particulier d'un solvant sélecté du groupe comprenant acétone, acétonitrile, chloroforme, dichlorométhane, diméthyl sulfoxyde, N,N diméthyl formamide et leurs mélanges.

10. Précurseur polycyclique de la formule générale (III) 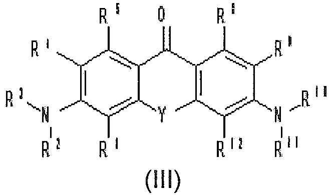
où R¹ R², R³, R⁴, R⁵, R⁸, R⁹, R¹⁰, R¹¹ et R¹² sont définis selon une des revendications 1 à 7 et
où Y représente CR¹⁵R¹⁶, R¹⁵ et R¹⁶ étant définis selon la revendication 1.

11. Méthode pour produire un précurseur polycyclique de la formule générale (III),
**caractérisé en ce que**
un précurseur polycyclique de la formule générale (IV) 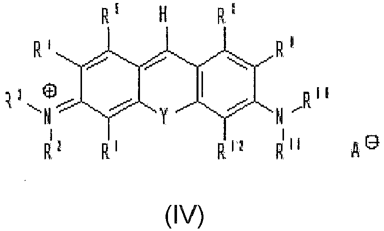 est transformé avec un oxydant
où R¹, R², R³, R⁴, R⁵, R⁸, R⁹, R¹⁰, R¹¹, R¹² Y et A⁻ sont définis selon une des revendications 1 à 7.

12. Application d'un composé polycyclique selon une des revendications 1 à 7 ou d'un précurseur polycyclique selon la revendication 10 pour la détermination qualitative ou/et quantitative d'un analyte dans un échantillon.

13. Application selon la revendication 12,
**caractérisée en ce que**
la détermination comprend un marquage de l'analyte par liaison covalente du composé polycyclique ou du précurseur polycyclique à l'analyte ou/et **en ce que** l'analyte est un biomolécule, en particulier un peptide, un polypeptide, un protéine, un nucléotide, un polynucléotide, un acide nucléique, un analogue de l'acide nucléique ou un haptène.
